(19) Europäisches Patentamt / European Patent Office / Office européen des brevets

(11) **EP 2 790 743 B1**

(12) # EUROPÄISCHE PATENTSCHRIFT

(45) Veröffentlichungstag und Bekanntmachung des Hinweises auf die Patenterteilung:
**06.06.2018 Patentblatt 2018/23**

(21) Anmeldenummer: **12816647.7**

(22) Anmeldetag: **16.12.2012**

(51) Int Cl.:
***A61L 27/50*** *(2006.01)* ***G03F 7/00*** *(2006.01)*
***G01N 13/02*** *(2006.01)*

(86) Internationale Anmeldenummer:
**PCT/DE2012/100382**

(87) Internationale Veröffentlichungsnummer:
**WO 2013/087073 (20.06.2013 Gazette 2013/25)**

(54) **IMPLANTAT MIT EINER MIKROSTRUKTURIERTEN OBERFLÄCHE SOWIE VERFAHREN ZU DESSEN HERSTELLUNG**

IMPLANT WITH A MICROSTRUCTURED SURFACE AND METHODS FOR THE PRODUCTION THEREOF

IMPLANT À SURFACE MICROSTRUCTURÉE ET SES PROCÉDÉS DE FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2011 DE 102011056549**

(43) Veröffentlichungstag der Anmeldung:
**22.10.2014 Patentblatt 2014/43**

(73) Patentinhaber: **Jennissen, Herbert P., Prof. Dr.**
**50858 Köln (DE)**

(72) Erfinder: **Jennissen, Herbert P., Prof. Dr.**
**50858 Köln (DE)**

(74) Vertreter: **Nobbe, Matthias**
**Demski & Nobbe**
**Patentanwälte**
**Mülheimer Strasse 210**
**47057 Duisburg (DE)**

(56) Entgegenhaltungen:

| | |
|---|---|
| **EP-A1- 1 254 673** | **EP-A2- 1 683 593** |
| **WO-A1-03/073045** | **WO-A2-2006/135755** |
| **DE-A1-102009 057 444** | **US-A1- 2008 195 216** |

- **H.P. JENNISSEN: "Ultra-hydrophilic Transition Metals as Histophilic Biomaterials", MACROMOL. SYMP., Bd. 225, 2005, Seiten 43-69, XP009083200,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 790 743 B1

## Beschreibung

Die Erfindung betrifft ein Implantat mit einer mikrostrukturierten Oberfläche sowie Verfahren zur Herstellung des Implantat mit einer mikrostrukturierten Oberfläche. Mittels des erfindungsgemäßen Verfahrens sind insbesondere Implantate mit anwenderspezifisch festgelegten Oberflächeneigenschaften herstellbar. So lassen sich mit dem erfindungsgemäßen Verfahren Implantate wie z.B. Zahnimplantate oder Endoprothesen, die sich durch ein besonders gutes Einwachsen am Implantationsort im Kiefer oder Extremitätenknochen auszeichnen, herstellen.

In den letzten Jahren ist es immer deutlicher geworden, dass die Rauhigkeit neben der Hydrophilizität und Hydrophobizität der Oberfläche eines Implantates eine der wichtigsten Rollen bei der Integration von Implantaten spielt. Die Rauhigkeit kann sowohl die Hydrophiliziät als auch die Hydrophobizität verstärken. So ist es im Stand der Technik bekannt, dass eine SLA-(sand-blasted acid etched)-Oberfläche ein wesentlich besseres Einwachsverhalten zeigt als die glatte maschinierte Form des Titans. Neben der SLA-Oberfläche mit einer Rauhigkeit gibt es Implantate mit einer TPS-(titanium plasma sprayed)-Oberfläche, die eine Rauhigkeit mit einem besseren Einheilverhalten zeigt.

Das Vorhandensein einer rauhen Oberfläche ist im Vergleich zu einer glatten Oberfläche immer mit einer Oberflächenvergrößerung verbunden. So können beispielsweise die SLA- und TPS-Oberflächen im Vergleich zu einer glatten Oberfläche eine 2-20 fach größere Oberfläche aufweisen, was sich besonders beim Einwachsen in Tier und Mensch positiv auswirkt.

Ein Nachteil rauher Oberflächen ist das Problem der Entfernung bei Implantatrevisionen. Den bisher hergestellten Implantaten ist insbesondere gemein, dass die nach außen weisenden Oberflächen des Gegenstandes in der Regel unregelmäßige Strukturen aufweisen, die insbesondere für die Verwendung der Gegenstände als Implantate das Einwachsverhalten beeinträchtigen und nicht positiv beeinflussen. Des Weiteren können sich Titanpartikel von der TPS-Oberfläche ablösen und ins Gewebe gelangen.

Hinzu kommt eine verbesserungswürdige Reproduktionsfähigkeit der so hergestellten Implantate, da sowohl nach dem SLA-Verfahren als auch nach dem TPS hergestellte Implantate eine gewisse statistische Breite der Oberflächeneigenschaften zeigen und daher eine genaueste Einhaltung der Verfahrensparameter in Abhängigkeit von dem Ausgangsmaterial zur Standardisierung der Implantate erforderlich ist.

Seitens des Erfinders wurden Überlegungen zur Verbesserung der Oberflächeneigenschaften angestellt, und es wurde herausgefunden, dass man mit einer Mikrostruktur eine optimal strukturierte Oberfläche des Implantates bereitstellen kann. Es wurde von dem Erfinder gezeigt, dass ein Reverse-Engineering zu einer Oberfläche mit gegenüber den beiden oben angegebenen SLA- und TPS-Oberflächen verbesserten Eigenschaften führt, wobei die verbesserten Oberflächen mit geringerem Gefahrenpotential hergestellt werden können.

Seitens des Erfinders wurde weiter herausgefunden, dass solche rauhen Implantatoberflächen mittels nasschemischer Verfahren und/oder durch Funktionalisierung mit hydrophilen organischen Molekülen weiter hyperhydrophil gemacht werden können, wie es weiter unten erläutert wird

Allerdings erfordert die Herstellung von solchen hyperhydrophilen Oberflächen zumeist den Einsatz von hocherhitzten Säuren und den entsprechenden Plasmakammern. Bei diesen hyperhydrophilen Oberflächen wurden bisher die dynamischen Kontaktwinkel mit Reinstwasser in Form des Vorrückwinkels ($\theta_V$) und des Rückzugswinkels ($\theta_R$) gemäß den Beobachtungen des Erfinders mit dem Wert Null ($\theta_V/\theta_R$ = 0°/0°) gemessen. In Wirklichkeit liegen die Kontaktwinkel im imaginären Bereich.

Die Erfindung ist daher gerichtet auf Implantate mit einer mikrostrukturierten Oberfläche, die, falls gewünscht, von einer zweiten kleineren Mikrostruktur und/oder von einer Nanostruktur überlagert ist, sowie Verfahren zu deren Herstellung, die die gewünschten hyperhydrophilen Oberflächeneigenschaften besitzen.

Eine derartige regelmäßige Mikrostruktur kann erfindungsgemäß mittels verschiedener Verfahren erzeugt werden. Dazu gehören strukturabtragende Verfahren wie auch strukturaufbauende Verfahren, die jeweils von der Beaufschlagung des Gegenstandes, oder von Pulver, mit energiereicher Strahlung Gebrauch machen.

Gemäß den Angaben in Mays (2007) " A new classification of pore sizes. Studies in Surface Science and Catalysis, 160, 57-62" zu Porengrößen können Strukturen/Rauhigkeiten wie folgt entsprechend klassifiziert werden:

Nanostrukturen: 0.1-100 nm
Mikrostrukturen: 0.1-100 μm
Millistrukturen: 0.1-100 mm

Dabei kann der Bereich 0.1-0.99 μm kann als "Submikrostruktur" bezeichnet werden.

Als strukturabtragendes Verfahren kann Laserabtragen erfindungsgemäß sehr selektiv eingesetzt werden, um einzelne Schichten vom Substrat ohne signifikante Beschädigung der darunterliegenden Schichten oder des Substrats zu entfernen. Die abgetragenen Strukturen können sowohl punkt- oder linienförmig sowie flächig ausgestaltet sein.

Als Struktur- oder Schicht-aufbauende Verfahren zur Herstellung von dreidimensionalen Gegenständen wie Implantaten sind erfindungsgemäß zu nennen: Rapid Prototyping, Rapid Tooling, Rapid Manufacturing, Lasersintern,

Lasermikrosintern und EBM.

Erfindungsgemäß kann als ein weiteres Verfahren zur Erzeugung von Mikrostrukturen das Lasermikrosintern verwendet werden. Dabei ist auch die Verarbeitung keramischer Pulver in hoher Qualität möglich.

Grundvoraussetzung für die Verfahren ist in der Regel, dass die Geometriedaten des Produktes dreidimensional vorliegen und als Schichtdaten verarbeitbar sind. Aus den vorliegenden CAD-Daten des Bauteils überführt man erfindungsgemäß die Daten in ein Datenformat, beispielsweise ein STL-Format, um die Oberfläche eines Rohlings mittels der vorgenannten Verfahren gezielt strukturieren oder den Rohling strukturiert aus Pulver aufbauen zu können.

Die bekannten Vorrichtungen, auch für Rapid-Prototyping-Verfahren, besitzen jeweils eine solche STL-Schnittstelle, die zur Bereitstellung geometrischer Information aus dreidimensionalen Datenmodellen dient.

Seitens des Erfinders wurde somit ein Verfahren entwickelt, bei dem sich mit regelmäßigen/periodisch wiederholenden Mikrostrukturen versehene Oberflächen dadurch erzeugen lassen, dass man die Oberfläche des Rohlings mit energiereicher Strahlung in einem oder mehreren Mustern beaufschlagt, das aus einer in einen STL-Datensatz überführten periodischen Funktion darstellbar ist, wobei entweder ein strukturaufbauendes Verfahren unter Verwendung von teilchenförmigem Material wie Metall-Pulver oder Keramik-Pulver oder ein strukturabtragendes Verfahren eingesetzt wird. Bei einem strukturaufbauenden Verfahren kann eine auf dem Rohling vorhanden Pulvermenge in einem oder mehreren Schritten mit der energiereichen Strahlung beaufschlagt werden und auf dem Rohling das Muster erzeugt werden.

Als weniger aufwändige Alternative bietet sich erfindungsgemäß ein strukturabtragendes Verfahren an, bei dem unter Abtragen von Oberflächenmaterial die gewünschte Struktur erzeugt wird.

Genauer betrifft die vorliegende Erfindung in einer Ausführungsform ein Verfahren zur Herstellung eines Implantats mit einer regelmäßig mikrostrukturierten Oberfläche mit Erhebungen und Vertiefungen, wobei der Abstand zwischen den Erhebungen im statistischen Mittel im Bereich von 1.0 bis 100 $\mu$m und die Profilhöhe der Erhebungen und Vertiefungen im statistischen Mittel (Ra-Wert) im Bereich von 1 bis 80 $\mu$m liegen, das die Schritte aufweist:

a) Bereitstellung eines Pulvers oder einer Pulvermischung aus einem sinterbaren Materialpulver auf einem Rohling;
b) Aufbringen einer Schicht des Materialpulvers auf der Oberfläche des Rohlings;
c) Beaufschlagen der Schicht des Materialpulvers mit energiereicher Strahlung in einem Muster, das aus einer in einen STL-Datensatz überführten periodischen Funktion darstellbar ist, so dass Materialpulver unter Ausbildung mindestens eines Teilbereiches des Musters auf zumindest einem Teilbereich der Oberfläche des Rohlings gesintert wird.

Bei dem Verfahren kann der Rohling aus Vollmaterial oder schichtweise über ein Sinterverfahren aus einem sinterbaren Materialpulver hergestellt sein.

Falls erforderlich, kann ein Bewegen oder Verschieben des Rohlings in axialer oder horizontaler Richtung und eine sukzessive Wiederholung der Schritte b) bis d) erfolgen, so dass ein an den ersten Teilbereich des Musters anschließender weiterer Teilbereich des Musters gesintert werden kann.

In einer Ausführungsform umfasst das Verfahren die sukzessive Wiederholung der Schritte b) bis c) bis zur vollständigen Bedeckung der Oberfläche mit dem gewünschten Muster.

Wie oben erwähnt, ist es neben dem Verfahren zum Aufbau eines Musters über Lasersintern oder EBM ebenso möglich, durch Laserabtragen eine gewünschte Oberflächenmikrostruktur zu erzeugen. So betrifft die Erfindung auch ein Verfahren zur Erzeugung eines Implantats mit mikrostrukturierten Oberfläche, das die Schritte aufweist:

a) Bereitstellen eines Rohlings;
b) Beaufschlagen des Rohlings mit energiereicher Strahlung zumindest teilweise in einem Muster, das aus einer in einen STL-Datensatz überführten periodischen Funktion darstellbar ist, so dass so dass unter Ausbildung mindestens eines Teilbereiches des Musters auf zumindest einem Teilbereich der Oberfläche der Rohling abgetragen wird.

Der erhaltene Rohling mit einer regelmäßig mikrostrukturierten Oberfläche des aufbauenden oder abtragenden Verfahrens kann einer Behandlung zur Erzeugung einer zweiten regelmäßigen Mikrostruktur unter Verwendung einer aus einer in einen STL-Datensatz überführten periodischen Funktion und/oder einer nasschemischen Behandlung zur Erzeugung einer Nanostruktur unterzogen wird.

Falls erforderlich, kann der Rohling in axialer oder horizontaler Richtung bewegt werden, und es kann Schritt b) gegebenenfalls mehrfach wiederholt werden, bis die Substratoberfläche zumindest in einem Teilbereich mit dem gewünschten mikrostrukturierten Muster versehen ist.

Die Erfindung umfasst somit auch ein Implantat, bei dem die hyperhydrophile Oberfläche unregelmäßig oder zumindest in Teilbereichen regelmäßig mikrostrukturiert ist.

Das Material des Rohlings kann ausgewählt werden aus der Gruppe der Metalle, der metallischen Legierungen, keramischer Materialien (z.B. Zirkonoxid), Gläsern und Polymeren (PEEK, Polyetheretherketone) sowie Kombinationen

davon.

Dabei besteht das Material des Rohlings insbesondere zur Verwendung als Implantat, bevorzugt aus einem Material, das aus der Gruppe der Metalle, der metallischen Legierungen und Kombinationen davon mit keramischen Materialien ausgewählt wird. Bevorzugt besteht das eingesetzte Implantatmaterial aus metallischen Materialien wie Reintitan oder metallischen Titanlegierungen, Chrom/Nickel/Aluminium/Vanadium/Kobalt-Legierungen (z.B. TiAlV4, TiAlFe2,5), Edelstählen (z.B. V2A, V4A, Chrom-Nickel 316L) oder aus einer Kombination davon mit keramischen Materialien wie Hydroxylapatit, Zirkonoxid, Aluminiumoxid, bei der das metallische Material als Verbundmaterial mit keramischem Material vorliegt. Die nichtmetallischen Materialien einschließlich der Polymere wie PEEK können aber auch allein ohne Kombination mit anderen Materialien eingesetzt werden.

Die so erhaltenen mikrostrukturierten Oberflächen können erfindungsgemäß durch eine nasschemische Behandlung mit beispielsweise Chromschwefelsäure weiter hydrophilisiert werden, wobei der Kontaktwinkel bei Benetzung mit Wasser dann gemäß der klassischen Messung und Auswertung nicht mehr messbar ist bzw. mit Null angegeben wird, jedoch nach dem neuen von dem Erfinder entwickelten Verfahren mit imaginären Zahlen angegeben werden kann..

Eine solche Behandlung kann beispielsweise so durchgeführt werden, dass man die Oberfläche des mikrostrukturierten Implantates mit einem Oxidationsmittel behandelt, indem man das bevorzugt entfettete Implantat in heißer Chromschwefelsäure - bevorzugt hat die Chromschwefelsäure dabei eine Dichte von mehr als 1,40 g/cm$^3$ - mit einer Temperatur von oberhalb von 200°C schockerhitzt, d.h. durch Eintauchen innerhalb von wenigen Sekunden auf die Temperatur der Chromschwefelsäure erhitzt, und dort bei dieser Temperatur für einen Zeitraum von 10 bis zu 90 Minuten, bevorzugt bis zu 60 Minuten, besonders bis zu 30 Minuten belässt und danach direkt nach der Entnahme das Implantat innerhalb eines Zeitraumes von unter einer Minute, bevorzugt innerhalb von wenigen Sekunden auf Raumtemperatur abkühlt. Dies kann vorzugsweise dadurch erfolgen, dass man das Implantat in konzentrierter Schwefelsäure mit einer Temperatur von 15°C bis 25°C durch Eintauchen abschreckt. Um Reste von Säure und, falls vorhanden, implantatfremde Metallionen, z.B. Chromionen, zu entfernen, wird die Oberfläche des Metallimplantates in mehreren Waschschritten (bis zu 15) mit destilliertem Wasser gewaschen. Falls auf der Oberfläche des Implantates danach noch Chromionen nachzuweisen sind, kann das Implantat mit einer Lösung eines Komplexierungsmittels solange behandelt werden, bis keine Metallionen mehr nachweisbar sind. Seitens der Erfinder wurde überraschenderweise herausgefunden, dass sich bei Verwendung von EDTA als Komplexierungsmittel die Lösung braunviolett violett verfärbt, wenn Chrom aus den Proben herausgelöst wird. Entsprechend schlagen die Erfinder für den Fall vor, dass die Proben so lange in 10% EDTA (1-3 x) bei pH 7, falls erforderlich auch in siedender EDTA-Lösung, gewaschen werden, bis keine Verfärbung durch Chromionen mehr auftritt.

Mittels dieses erfindungsgemäßen Verfahrens ist somit ein Implantat mit einer hyperhydrophilen Oberfläche erhältlich, das gemäß einer weiteren Ausbildung eines Verfahrens gemäß der EP 2.121.058 lagerfähig gemacht werden kann.

Dort haben die Erfinder Versuche durchgeführt, die im Vergleich zu den im Stand der Technik bekannten Lehren überraschende Ergebnisse ergaben. Aufgrund der Aufwendigkeit von Nassverpackungen zur Konservierung hydrophiler und ultrahydrophilen Oberflächen auf Implantaten, die bei den erfindungsgemäßen ultrahydrophilen Metallimplantaten überraschenderweise auch bei höheren Salzkonzentrationen von mehr als 0,5 M/l lagerstabile Implantate ohne Benetzbarkeitsverlust ermöglichen, wurde auch nach flüssigkeitsfreien Verpackungsmethoden gesucht. Dabei wurde gefunden, dass hyperhydrophile Oberflächen, auf denen man Salzlösungen evaporieren ließ, stabil ebenfalls gegen den Benetzbarkeitsverlust wurden. Die Evaporierung kann unter Schutzgas oder in atmosphärischer Luft erfolgen, wobei letztere wegen der Einfachheit standardmäßige Verwendung gefunden hat.

Auf der so behandelten Oberfläche bildete sich nach Evaporation eine feine makroskopisch unsichtbare "Exsikkationsschicht", die erfindungsgemäß sowohl die Ultrahydrophilizität als auch die Hyperhydrophilizität stabilisiert und schützt. Allgemein lassen sich erfindungsgemäß Neutralsalzlösungen in Lösung eines einzelnen Salzes oder auch verschiedener Salze in einer Konzentration und Menge verwenden, die gegenüber der ultrahydrohilen Oberfläche inert ist und ausreichend ist, nach dem Abdampfen die Oberfläche des Implantates mit der Exsikkationsschicht zu bedecken. Die Evaporation kann durchgeführt werden, wenn sich das Implantat in der Lösung aus Neutralsalz befindet, oder dann, wenn das Implantat aus der Lösung entnommen wurde und so nur mit einer dünnen Schicht dieser Lösung bedeckt ist. Entsprechendes gilt auch für die hier beschriebenen hyperhydrophilen Implantate.

Die Erfindung umfasst daher auch ein Verfahren, das neben den zuvor beschriebenen Schritten zur Herstellung des Implantats den zusätzlichen Schritt umfasst, dass die erhaltene Oberfläche mit Hilfe einer Lösung nicht-flüchtiger Substanzen wie Salzen, organischen Lösungsmitteln, die nicht mit der Oberfläche wechselwirken, oder einer salzhaltigen Exsikkationsschicht zum Schutz der Oberfläche des Substrates gegenüber der Abnahme durch Alterung oder durch Sterilisierungsverfahren (z.B. Gammasterilisierung) geschützt, stabilisiert und langzeitlagerfähig gemacht wird.

Die Erfindung ist auch gerichtet auf ein Implantat mit einer mikrostrukturierten hyperhydrophilen Oberfläche mit Erhebungen und Vertiefungen, wobei der Abstand zwischen den Erhebungen im statistischen Mittel im Bereich von 1 bis 100 μm und die Profilhöhe der Erhebungen und Vertiefungen im statistischen Mittel (Ra-Wert) im Bereich von 1

bis 80 $\mu$m liegen, wobei zumindest einer der beiden dynamischen Kontaktwinkel ($\theta_V$ und $\theta_R$) im hyperhydrophilen Bereich mit $\frac{\Delta F}{P \cdot Y} > 1{,}0$ bis 2,15 ($\theta_{ai}$ > 0,0i° - 80i° ), besonders mit $\frac{\Delta F}{P \cdot Y} > 1{,}0$ bis 1,0619 ($\theta_{ai}$ > 0,0i° - 20i°) liegt.

Die Erfindung umfasst ferner ein Implantat wie zuvor definiert, bei dem die erste Mikrostruktur mit ersten Erhebungen und Vertiefungen von einer zweiten Mikrostruktur mit zweiten Erhebungen und Vertiefungen überlagert ist, wobei der Abstand zwischen den zweiten Erhebungen im statistischen Mittel im Bereich von 0,1 bis 10 $\mu$m liegt und die Höhe der zweiten Erhebungen und Vertiefungen im statistischen Mittel (Ra-Wert) im Bereich von 0,1 bis 10 $\mu$m liegt.

Bevorzugt beträgt Abstand zwischen den zweiten Erhebungen im statistischen Mittel im Bereich von 0,1 bis 5 $\mu$m und die Höhe der zweiten Erhebungen im Bereich von 0,1 bis 5 $\mu$m.

Die Mikrostruktur mit den ersten Erhebungen und Vertiefungen oder die Mikrostruktur mit den zweiten Erhebungen und Vertiefungen können von einer Nanostruktur überlagert sein, die durch eine nasschemische Behandlung, z.B. durch Säureätzung, wie weiter unter erläutert erzeugt werden kann.

Von Vorteil ist es, wenn die Oberfläche eine regelmäßige erste Struktur aufweist und die Abstände und Höhen der ersten Erhebungen in den zuvor definierten Grenzen liegen. Diese erste Mikrostruktur wird vorzugsweise dadurch erzeugt, dass die Oberfläche des Rohlings mit energiereicher Strahlung in einem Muster, das aus einer in einen STL-Datensatz überführten periodischen Funktion darstellbar ist, beaufschlagt wird. Diese periodische Funktion ist vorzugsweise eine trigonometrische Grundfunktion $A_R(x)$ die ausgewählt wird aus:

$$A_R(x) = (\sin(x) \,, \qquad (1)$$

$$A_R(x) = \frac{4a}{\pi}(\sin(x) + \frac{1}{3}\sin[3x] + \frac{1}{5}\sin(5x) + \frac{1}{7}\sin(7x) + \frac{1}{9}\sin(9x) + .....) \,, \qquad (2)$$

$$A_R(x) = \frac{4a}{\pi}\left(\sin(x) - \left(\frac{1}{3}\right)^2 \sin(3x) + \left(\frac{1}{5}\right)^2 \sin(5x) - \left(\frac{1}{7}\right)^2 \sin(7x) + \left(\frac{1}{9}\right)^2 \sin(9x) + .....\right) , \qquad (3)$$

$$A_R(x) = \frac{2a}{\pi}(\sin(x) - \frac{1}{2}\sin[2x] + \frac{1}{3}\sin(3x) - \frac{1}{4}\sin(4x) + \frac{1}{5}\sin(5x) + .....) \,, \qquad (4)$$

oder Ableitungen davon.

Eine optionale, die erste Mikrostruktur überlagernde zweite Mikrostruktur wird vorzugsweise dadurch erzeugt, dass die Oberfläche des Rohlings mit energiereicher Strahlung in einem Muster, das aus einer in einen STL-Datensatz überführten periodischen Funktion darstellbar ist, beaufschlagt wird. Diese periodische Funktion kann vorzugsweise eine wie zuvor angegeben trigonometrische Grundfunktion $A_R(x)$ sein, die mit anderen Variablen zu einer kleineren "Wellenlänge" und "Amplituden" der Mikrostruktur führt.

Der erfinderischen Entwicklung des Erfinders liegt die Erkenntnis zugrunde, dass als ein wichtiger Parameter der profilometrische arithmetische Mittelwert der Rauhigkeit (Ra-Wert) Auskunft über die Topographie der Oberfläche gibt.

Hierzu wird gemäß den Überlegungen des Erfinders eine Referenzlinie auf einer Substratoberfläche so gelegt, dass die Fläche der Berge und Täler gleich groß wird. Dabei wird Ra als das arithmetische Mittel der absoluten Abweichungen der Profilhöhen nach oben und unten in $\mu$m definiert. Folgende vereinfachte Gleichung beschreibt den Ra-Wert:

$$R_a = (z_1 + z_2 + z_3 + ...\ z_n)/n \qquad [\mu m] \qquad (5)$$

Der Absolutwert der Profilhöhe (positive oder negative Höhe auf der y-Achse) bezogen auf die Profilreferenzlinie wird z genannt. L ist eine definierte Meßlänge (Fenster) entlang der x-Achse. Idealisiert entspricht ein solches Oberflächenprofil einer regelmäßigen Sinusschwingung mit den Extremwerten $\pm$ z in Abweichung von der Referenzlinie (Nullinie) (siehe **Fig. 2A**). Neben dem Ra-Wert wird eine zweite topographische Größe als maximale Profilhöhe Ry (= Summe aus höchster Profilkuppe und tiefstem Profiltal) ebenfalls in $\mu$m definiert. Schließlich ist zu berücksichtigen, dass Oberflächen mit identischen Ra-Werten nicht identische Oberflächenprofile besitzen können.

Ein weiterer Rauhigkeitsparameter ist der dimensionslose mikroskopische Rauhigkeitsfaktor $r_m$:

$$r_M = \frac{tatsächliche\,Oberfläche}{geometrische\,Oberfläche} = \frac{A'}{A} \qquad (6)$$

wobei A' die gemessene vergrößerte Oberfläche im Vergleich zur errechneten geometrischen Oberfläche A darstellt. Der mikroskopische Rauhigkeitsfaktor r, der in der Regel mit Hilfe eines Laser Scanning Mikroskopes (LSM) aufgenommen wird, gibt Auskunft über die mikroskopische Größenzunahme der Oberfläche durch die Rauhigkeitsvergrößerung.

In Zellkulturen hat sich gezeigt, dass regelmäßige Strukturen ohne scharfe Kanten auf der Biomaterialoberfläche von Zellen bevorzugt werden. Ferner hat sich gezeigt, dass sich in Oberflächen mit enghalsigen Poren, wie sie z.B. bei TPS-Oberflächen vorkommen können, Biofilme bilden, die eine Implantantlockerung hervorrufen können. Ziel ist es daher eine Oberfläche ohne solche Poren zu erstellen.

Die Strukturierung der Substrat-Oberfläche in dem Muster, das aus einer in einen STL-Datensatz überführten periodischen Funktion darstellbar ist, verleiht dem Substrat Eigenschaften, welche durch Variation bestimmter Parameter, wie beispielsweise Rauhigkeit (Ra-Werte), Periodizitätswert, mikroskopischer Rauhigkeitsfaktor $r_M$, Abstand zwischen den Erhebungen oder maximale Profilhöhe Ry beeinflusst werden können. Erfindungsgemäß sind Substrate und Verfahren zur Herstellung von Substraten bevorzugt, die einen Rauhigkeitsparameter Ra im Bereich von 1-250 $\mu$m, bevorzugt zwischen 1 und 80 $\mu$m und besonders bevorzugt zwischen 2 und 30 $\mu$m besitzen oder erzeugen. Erfindungsgemäß sind Substrate und Verfahren zur Herstellung von Substraten bevorzugt, die einen Periodizitätswert n($\lambda$/2) im Bereich zwischen 1 und 100 $\mu$m, bevorzugt zwischen 10 und 60 $\mu$m und besonders bevorzugt zwischen 2-30 $\mu$m besitzen oder erzeugen. Erfindungsgemäß sind Substrate und Verfahren zur Herstellung von Substraten bevorzugt, die einen mikroskopischen Rauhigkeitsfaktor $r_M$ im Bereich zwischen 2 und 50 besitzen oder erzeugen. Erfindungsgemäß sind Substrate und Verfahren zur Herstellung von Substraten bevorzugt, die einen Abstand zwischen den Erhebungen im statistischen Mittel im Bereich von 1 bis 100 $\mu$m besitzen oder erzeugen. Erfindungsgemäß sind Substrate und Verfahren zur Herstellung von Substraten bevorzugt, die als maximale Profilhöhe Ry in einem Bereich von 2 bis 500 $\mu$m besitzen oder erzeugen.

Die Idee zur Herstellung von Implantaten mit homogener und definierter Rauhigkeit hat der Erfinder über die Betrachtung der Rauhigkeit als Sinuskurve entwickelt. Dies ist in **Fig. 2A** gezeigt, in der ein Oberflächenprofil mit Hilfe einer Sinuskurve beschrieben wird. Die Kurve läßt sich mit einer Wellenlänge von $\lambda$ = 32 $\mu$m sowie mit einer Amplitude (= Ra-Wert von 3.13 $\lambda$/2, 50 $\mu$m) beschreiben.

Für die Variation der Parameter $\lambda$ und Profilhöhe gilt nach Gleichung 1 die definierte Profilfunktion:

$$z = A_R\,(x) = (\sin(\frac{2\pi}{\lambda}x) \square P \qquad (7)$$

wobei x die unabhängige Variable, $\lambda$ die Wellenlänge und P die Profilhöhe ist. Für die definierte Profilfunktion in Fig. 2A gilt die Gleichung:

$$z = A_R\,(x) = (\sin(\frac{2\pi}{32}x) \square 50\,. \qquad (7a)$$

Auf diesem Wege ist es möglich, alle gewünschten Oberflächenprofile über die Grundgleichungen 1-4 darzustellen.

Die Verallgemeinerung dieses Prinzips ist in **Fig. 2B-D** mit den zugehörigen trigonometrischen Grundfunktionen gezeigt, wo gezeigt ist, dass neben einem sinusoiden Profil auch beispielsweise ein Rechteckprofil, ein Dreieckprofil und ein Sägezahnprofil erzeugt werden kann. Alle Profile, die mit trigonometrischen Funktionen oder Reihen beschrieben werden können, sind mittels des erfindungsgemäßen Verfahrens herstellbar. Mit diesem mathematischen Werkzeug lassen sich auch die gesuchten Parameter mit Ra-Werten im Bereich von 1-80 $\mu$m und $r_m$-Werten im Bereich von 2-50 in CAD-System entwerfen und für die Fertigung bestimmen. Somit lassen sich solche Oberflächenstrukturen bis in den Mikrometerbereich mit Hilfe des selektiven Elektronenstrahlschmelzens (SEBM), des selektiven Laserschmelzens oder der selektiven lasergestützten Fertigung herstellen.

In **Fig. 3** ist gezeigt, daß die Oberflächenrauhigkeit auf einer Fläche mit zwei sinosoiden Profilen (Koordinaten: X/Z und YZ) beschrieben werden kann. Es wird ferner gezeigt, wie mit Hilfe von Rechteckfunktionen eine Oberfläche mit massivquadratischen Profilen konstruiert werden kann. In **Fig. 3A** ist eine Einheitszelle mit einer Wellenlänge von

$\lambda/2 = 32\ \mu m$ dargestellt, d.h. alle 32 $\mu$m (Wellenberg) kommt ein Rechteckprofil das in der X- und Y- Koordinate den gleichen Abstand von $\lambda/2 = 32\ \mu m$ (Wellental) besitzt. Die Amplitude (Z-Achse) beträgt für beide sinusioide Funktionen den identischen Wert von 80 $\mu$m ($5\lambda/4$), was dem Ra-Wert entspricht. Die ganze Oberfläche lässt sich in Einheitszellen ($3\lambda$ x $3\lambda$) aufteilen mit einer Größe von 192 x 192 $\mu$m. Eine Einheitszelle besitzt hier 9 Profile (**Fig. 3A**). Es lassen sich auch mehrere Wellenlängen kombinieren z.B. in X-Richung $\lambda/2 = 32$ und in der Y-Richtung $\lambda/2 = 32$ und 16 $\mu$m alternierend (**Fig. 3B**). Hierdurch lassen sich jetzt in der gleichen Einheitszelle (192 x 192 $\mu$m) 12 Rechteckprofile plazieren (**Fig. 3B**).. Bei den Rechteckprofilen lassen sich die Oberflächen wie folgt berechnen:

$$F_{Profil} = 2(xy + xz + yz) - (xy) \qquad (3)$$

wobei x, y, und z die angegebene Koordinaten sind und die Grundfläche (xy), auf der das Profil steht, subtrahiert werden muß. Für aus **Fig. 3A und B** dargestellten und in **Tab. 2** berechneten Flächen (Wellenberge) gilt dann.

$$F_{Profil} = 2\ [(\lambda/2)(\lambda/2)+(\lambda/2)(5\lambda/4)+(\lambda/2)(5\lambda/4)]-[(\lambda/2)(\lambda/2)] \qquad (4)$$

Es müssen dann jedoch noch die freien Flächen inklusive der Wellentäler berechnet werden:

$$F_{Bodenfläche} = 3\ (\lambda_Y/2xL_{EZ}) + 9x(\lambda_X/2)^2 \quad (5)$$

(bei gleich großen Abständen)
oder

$$F_{Bodenfläche} = 3(\lambda_Y/2 \text{ x } L_{EZ}) + 12(\lambda_{x1} \text{ x } \lambda_{x2}) \ (6)$$

(bei unterschiedlichen Abständen)
wobei $L_{EZ}$ die Länge des Einheitsquadrates darstellt. Weitere oberflächenrelevanten Muster und Profile, die rechnerisch nicht berücksichtigt wurden, sind in **Fig. 3C** und **Fig. 3D** dargestellt.

Wie die Berechnungen in **Tab. 2** zeigen, lassen sich auf diesem Wege sehr einfach Oberflächen mit Ra-Werten und $r_m$-Werten konstruieren. So ergibt sich für die **Oberfläche A** ($\lambda/2 = 32\ \mu m$) in **Tab. 2** (siehe auch **Fig. 3A)** ein $r_m$-Wert von 6.0 bei einem Ra-Wert von 80 $\mu$m. Vermindert man nun den Ra-Wert auf 35 $\mu$m so sinkt der $r_m$-Wert auf 3.2 **(Oberfläche B).** Vermindert man die Wellenlänge auf $\lambda/2 = 8\ \mu m$ bei einem Ra-Wert von 80 $\mu$m dann erhält man tatsächlich eine Oberfläche mit einem $r_m$-Wert = 22.6 **(Oberfläche E).** Senkt man nun den Ra-Wert auf 35 $\mu$m, so erhält man einen $r_m$-Wert = 11.3, was auch noch beachtlich ist **(Oberfläche F).**

Bei Verwendung von mehreren verschiedenen Wellenlängen lassen sich (siehe **Fig. 1B,** 12 Profile/Einheitszelle) wesentlich höhere Oberflächenwerte erhalten **(Oberflächen G und H).** Eine interessante Möglichkeit zur Erhöhung der Oberflächengröße wäre die Verwendung von Hohlzylindern oder sternförmigen Profilen (siehe **Fig. 3D**). Zusammenfassend zeigt Tab. 2 eindeutig, dass man mit dem trigonometrischen Ansatz und Parametern im $\mu$m Bereich Ra-Werte im Bereich von 2-80 $\mu$m und $r_m$-Werte im Bereich von $r_m$ = 3.2-22.6 $\mu$m erreichen kann.

Durch die verfügbaren Technologien, wie das selektive elektronenstrahlschmelzen (SEBM), selektives Laserschmelzen oder Lasergestützte Fertigung (Lasergravur, rapid manufacturing) lassen sich somit die Oberflächen aller Biomaterialfestkörper auf diese Art und Weise durch Abtragen oder aus Pulvern aufbauend herstellen. Dabei ist eine Mikrostruktur auch bei einer Auflösung unter 10$\mu$m möglich. Die Erfindung zeigt, dass eine computergesteuerte Herstellung solcher Implantatoberflächen möglich ist.

Vorteile des erfindungsgemäßen Verfahrens sind somit:

- Bessere Verträglichkeit durch Homogenität der Oberfläche
- Große Oberflächen über Oberflächenvergrößerungen 20-40fach
- Vergrößerung der Oberflächenkapazität für Proteine und Pharmaka um das 20-40fache
- Verstärkung der Ultrahydrophilie
- Vermeidung von Infektionen
- Pharmakonreservoir in Hohlzylinderprofilen
- Computergestützte Fertigung (Laser und Elektronenstrahltechnologie)

Gemäß der Erfindung können alle Oberflächenstrukturen als trigonometrische Funktionen beschrieben werden,

die folglich direkt als 3D-Vektorgrafiken in AutoCAD dargestellt und simuliert werden können. Die Variationsmöglichkeiten zur Gestaltung der Oberfläche sind erfindungsgemäß vielfältig, und die Herstellung einheitlich-strukturierter, regelmäßiger sowie hochkomplexer Oberflächen ist mathematisch vordefiniert und auf jede Oberfläche eines beliebigen Bauteiles übertragbar. Die Umsetzung der Daten von der trigonometrischen Funktion beispielweise für ein Rapid-Prototyping-Verfahren und eine RP-Vorrichtung ist schematisch in Schema 1 dargestellt.

**Schema 1**

**Trigonometrische Funktionen**

↓

**Visual Basic for Applications® (VBA)**

↓

**AutoCAD® Code Script**

↓

**Script-Import in AutoCAD®**

↓

**3D CAD Modell in AutoCAD®**

↓

**Modell-Export als STL-Surface File**
(Format: triangulated surface mesh as binary and ASCII file)

↓

**STL-gesteuerte RP-Machine**

↓

**Fertiges Bauteil mit definierter Oberfläche**

Auf den erfindungsgemäßen optional mit zweiten Mikrostrukturen und/oder Nanostrukturen überlagerten Mikrostrukturen können in einem weiteren Schritt Peptide wie Knochenwachstumsfaktoren kovalent oder mittels physisorptiver oder chemisorptiver Bindung, vermutlich aufgrund hydrophiler Wechselwirkungen auf dem Implantatmaterial immobilisiert werden. Eine adsorptive Bindung ist auch nach einer kovalenten Modifikation der Oberfläche mit Aminopropyltriethoxysilan (APS) möglich (Tabelle 1). Dadurch wird ermöglicht, eine chemotaktisch wirkende und/oder biologisch aktive, bei kovalenter Bindung eine sogenannte juxtakrine, Implantatoberfläche auszubilden, die zur Ansiedlung, Proliferation und Ausdifferenzierung von Knochenzellen führt. So lassen sich sogenannte biologisch aktive Implantate bereitstellen, die bei von der Oberfläche freigesetzten Molekülen auch auf eine Entfernung von 500 bis 1000 μm eine chemotaktische Wirkung auf Zellen, im Falle von BMPs auf Osteoblasten, zeigen.
Bevorzugt wird die ausreichende Beladung der hydrophilierten Metalloberfläche dadurch erzielt, dass man die Peptide in einer physiologischen Pufferlösung in einer Konzentration, die ausreicht, eine Beladung von mehr als 200 $ng/cm^2$, bevorzugt mehr als 500 $ng/cm^2$, und mehr bevorzugt von mehr als 1000 $ng/cm^2$ des Peptids auf der Oxidoberfläche des Metallimplantates zu erzielen, aufbringt.
In der Regel wird diese Beladung mit einer physiologischen Pufferlösung von Peptiden in einer Konzentration von mehr als 1 μg/ml, bevorzugt mehr als 200 μg/ml Pufferlösung erzielt.
Erfindungsgemäß sind die Peptide Biomoleküle, die vorteilhaft für die Biokompatibilität des Implantates sind, indem sie einer möglichen Abstoßung des Implantates entgegenwirken und/oder das Einwachsen des Implantates fördern.
Wie oben erwähnt, können als Peptide bevorzugt Proteine aus der Klasse der TGF-Proteine, insbesondere das Knochenwachstum fördernde Proteine aus der Klasse der Knochenwachstumsfaktoren "Bone Morphogenic Proteins", oder der Klasse der Gefäßwachstumsfaktoren wie VEGF oder Angiotropin oder auch Ubiquitin verwendet werden. Unter der Bezeichnung "Transforming Growth Factor" (TGF) sind insbesondere die Gruppe (Subgruppe) der (i) "Transforming Growth Factors beta" (TGF-β) sowie die Gruppe (Subgruppe) der (ii) Bone Morphogenetic Proteine (BMP) zu

verstehen. Letztere sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von alkalischer Phosphatase, Hormonrezeptoren, knochenspezifischer Substanzen wie Kollagen Typ 1, Osteocalcin, Osteopontin, Osteonectin, Bone Sialoprotein (BSP) und schließlich die Mineralisation.

Vorteilhaft kann zur Immobilisierung ein Protein dieser Klasse allein, in Kombination mit weiteren Mitgliedern dieser Klasse oder auch zusammen mit Biomolekülen wie Proteinen anderer Klassen oder niedermolekularen Hormonen oder auch Antibiotika zur Verbesserung der Immunabwehr eingesetzt werden. Dabei können diese weiteren Moleküle auch über im physiologischen Milieu spaltbare Bindungen auf der Oberfläche immobilisiert werden.

Die Erfindung wird anhand der beigefügten Figuren weiter erläutert. Dabei zeigen:

Fig. 1: REM-Bilder typischer weitverbreiteter und erfolgreicher rauher Oberflächen in der Zahnheilkunde und Orthopädie mit A und B SLA-Oberfläche (sandblasted acid etched); C und D TPS-Oberfläche (Titan-Plasma-Spray Verfahren), und Insert Fig. 1C: Querbruchkante der TPS-Oberfläche. Der Pfeil weist auf den Fusionsspalt zwischen TPS-Schicht aus Reintitan und dem Grundmaterial aus Titanlegierung (Ti-6Al-4V);

Fig. 2: Grundformen von Oberflächenerhebungen (Profile) in der Seitenansicht. Dabei sind Möglichkeiten für die Spitze des Profils rund, flach und spitz bei $\lambda$ = 32 μm; z = 50 μm.
Die trigonometrischen Gleichungen, die die Profile beschreiben, sind unter den Figuren angegeben. Alle Möglichkeiten der Rauhigkeit können über solche Fourierreihen beschrieben werden.
Zugehörige 3D-Grundformen sind: A. Hyperboloid; B. Quader; C. Pyramide; D. asymmetrische Pyramide;

Fig. 3: Einheitszellen mit den jeweiligen zugehörigen Rechteckfunktionen sowie einige Profile (A & B) und Anordnungsmuster (C) in Querschnittsansicht mit:

A. Einheitszelle 192 μm x 192 μm mit 9 Profilen ($\lambda$x und $\lambda$y = 64 μm, z = Ra = 80 μm) (siehe Tab. 2, Oberfläche A)
B. Einheitszelle 192 μm x 192 μm mit 12 Profilen ($\lambda$x 64 μm und $\lambda$y = 32 μm, z = Ra = 80 μm); (siehe Tab. 2, Oberfläche G)
C. Anordnungsmuster für Profile
D. Profile mit unterschiedlichen Flächenwerten

3D-Grundform A-C: Quader

Fig. 4: Kombination einer ersten Mikrorauhigkeit ($\lambda$ = 64 μm) mit einer zweiten Mikrorauhigkeit ($\lambda$ = 7.1 μm). Durch die gezeigte zweite Mikrorauhigkeit wird die Oberfläche der Makrorauhigkeit um den Faktor 2.25 (1.5 x 1.5) vergrößert.

Fig. 5: Bestimmung der statischen (A) und dynamischen (B) Kontaktwinkel auf einer superhydrophoben unmodifizierten TPS Oberfläche mit ultrareinem Wasser.

Fig. 6: Bestimmung der dynamischen Kontaktwinkel auf einer Oberfläche nach chemischer "Umschaltung" vom superhydrophoben in den hyperhydrophilen Zustand.

Fig. 7: Darstellung der Wilhelmy-Funktionen in der undefinierten Region von Fig. 6 als imaginäre Kontaktwinkel in Abhängigkeit von der Eintauchtiefe.

Ohne Veränderung der o.g. Mikrostruktur wurde die mikrostrukturierte Oberfläche weiter nasschemisch nanostrukturiert und mit Aminopropyltriethoxysilan für die Adsorption von BMP-2 umgesetzt. Für die Berechnung der Monolayerbedeckung mit BMP-2 wurde ein "Footprint" des BMP-2 von 20 $am^2$ (1 μg BMP-2 ~ 4.6 $cm^2$ für eine monomolekulare Bedeckung der Oberfläche eingesetzt. Unter den gegebenen Bedinungen stimmt der mit BMP-2 bestimmte $r_m$-Wert gut mit den LSM-bestimmten $r_m$-Werten gut überein. Die erhaltenene Adsorptionswerte sind in Tabelle 1 angegeben.

Tabelle 1

| **Oberfläche** | **Ra μm** | **$r_M$ (A'/A)** | BMP-2 Adsorption **(APS-Oberfläche) ng/cm²** | | **$r'_M$ (A'/A)** |
|---|---|---|---|---|---|
| | | **LSM** | pro geometr. Fläche **(A)** | pro tatsächl. Fläche **(A')** | **BMP-2** |
| SLA | ~2-3 | 2.5 | 394 ± 66 (6) | 157 ± 27 (6) | 1.8 |

(fortgesetzt)

| **Oberfläche** | **Ra µm** | **$r_M$ (A'/A)** | BMP-2 Adsorption **(APS-Oberfläche) ng/cm²** | | **$r'_M$ (A'/A)** |
|---|---|---|---|---|---|
| | | **LSM** | pro geometr. Fläche **(A)** | pro tatsächl. Fläche **(A')** | **BMP-2** |
| TPS | 30.0 ± 4.4 (4) | 20 | 5221 ± 293 (6) | 261 ± 15 (6) | 23.9 |
| Datenformat: ξ ± S.D. | | | | | |

Erfindungsgemäß können die Oberflächen-Mikrostrukturen unter Anwendung der trigonometrischen Funktionen wie gewünscht hergestellt werden. Dazu können die Rauhigkeitsparameter für ein Rechteckprofil mit einer Profilhöhe von 35 und 80 µm mit den Werten wie aus Tabelle 2 ersichtlich verwendet werden.

Tabelle 2

| Oberfläche | $\lambda_x$ | $\lambda_y$ | X λ/2 | Y λ/2 | $F_{Profil}$ | Profile/ EZ | Ra | Ry | $r_m$ |
|---|---|---|---|---|---|---|---|---|---|
| | µm | µm | µm | µm | µm² | | µm | | |
| **A** | 64 | 64 | 32 | 32 | 21504 | 9 | 80 | 160 | **6.0** |
| **B** | 64 | 64 | 32 | 32 | 9 984 | 9 | 35 | 70 | **3.0** |
| **C** | 32 | 32 | 16 | 16 | 10 496 | 36 | 80 | 160 | **11.6** |
| **D** | 32 | 32 | 16 | 16 | 4 266 | 36 | 35 | 70 | **6.9** |
| **E** | 16 | 16 | 8 | 8 | 5 248 | 144 | 80 | 160 | **22.6** |
| **F** | 16 | 16 | 8 | 8 | 2 304 | 144 | 35 | 70 | **10.3** |
| | | | | λ/2 + λ/4 | | | | | |
| **G** | 64 | 32 | 32 | 32 + 16 | 21 504 | 12 | 80 | 160 | **7.8** |
| **H** | 16 | 8 | 8 | 8 + 4 | 2 304 | 192 | 35 | 70 | **13.6** |

Die Oberflächen A und G sind in **Fig. 3 A** und **3B** dargestellt. Die Fläche der Einzelzelle (EZ) beträgt 36 864 µm².

Mithilfe des erfindungsgemäßen Verfahrens können somit Substrate wie Implantate mit definierten Oberflächenstrukturen bereitgestellt werden, die direkt nach der lasertechnischen Herstellung hyperhydrophil sind. Ist die Oberfläche nicht ausreichend hyperhydrophil, kann sie mit Hilfe eines chemischen Hydrophilierungsverfahrens weiter hyperhydrophiliert werden. So führen diese Oberflächenstrukturen, die auch Implantate tragen können, zu besonderen Benetzungseigenschaften, die erfindungsgemäß als hyperhydrophile Oberflächen bezeichnet werden. Zu solchen chemischen Hydrophilierungsverfahren zählen nasschemische Methoden wie Säureätzungen als auch durch kovalente oder nicht-kovalente Bindung von hoch-hydrophilen organischen Molekülen wie Polyethyenglycol (PEG), poly(2,3-dihydroxypropyl methacrylate) (PDHMA) oder poly[2-(methacryloyloxy) ethyl phosphorylcholine] (PMPC) funktionalisierte strukturierte Oberflächen, wobei PDHMA und PMPC eine zwitterionische Struktur besitzen. Eine kovalente Kopplung kann beispielsweise über entsprechende Triethoxysilanderivate von PEG, PDHMA und PMPC erfolgen.

Wie im Stand der Technik bekannt, werden in der vorliegenden Anmeldung zur Charakterisierung der Benetzbarkeit oder hydrophilie Oberflächen mit dynamischen Kontaktwinkeln mit einem Wert von $0 < \theta < 10°$ als ultrahydrophil bezeichnet, während Oberflächen mit den erfindungsgemäß bestimmbaren Kontaktwinkeln in Form von imaginären Kontaktwinkeln mit einem Wert von $\theta > 0i$ bis $1,4i$ rad als hyperhydrophil bezeichnet.

Üblicherweise erfolgt die Messung der Hydrophilizitätseigenschaften einer Oberfläche auf Basis der Bestimmung des Kontaktwinkels. Die Einführung des Kontaktwinkels θ durch Thomas Young vor über 200 Jahren eröffnete dabei grundsätzlich den Weg zum Verständnis der Benetzbarkeit durch die Einführung der Young-Gleichung :

$$\gamma_{sv} = \gamma_{sl} + \gamma_{lv} \cos \theta_o \qquad (7)$$

wobei $\gamma_{sv}$ , $\gamma_{sl}$ and $\gamma_{lv}$ die Grenzflächenspannungen der sich berührenden Phasengrenzen von Flüssigkeit (*l*), Festkörper (*s*) und Dampf-/Gasphase (*v*) darstellen, mit $\theta_0$ als Gleichgewichts-Kontaktwinkel z.B. eines sitzenden Tropfens. Die Young-Gleichung, die für eine vollkommen glatte Oberfläche gilt, ist nicht einfach zu lösen, da in der Regel nur $\gamma_{lv}$ and $\theta_0$ meßbar sind.

Es ist zu beachten, das die Young Gleichung für Kontaktwinkel für ideale, glatte, impermeable Oberflächen im

thermodynamischen Gleichgewicht gilt. Kontaktwinkel auf realen, rauen Oberflächen werden dagegen mit dem Attribut "apparent" bezeichnet, um sie vom Young-Kontaktwinkel auf einer idealen Oberfläche zu unterscheiden.

Davon ausgehend erfand Ludwig Wilhelmy (Ann. Phys., 119, 177-217) etwa 60 Jahre später die Wilhelmy-Waage, in dem er die Tensiometrie mit Kontaktwinkelmessungen verband. Bei Kraftmessungen mit Hilfe der Wilhemy-Waage wird die Probe unter Messung der Kraft in Reinstwasser eingetaucht und herausgezogen. Der Kontaktwinkel wird dann aus der Kraft des Ein- und Auftauchens der Probe nach der bekannten Wilhelmy-Gleichung berechnet:

$$F = P \gamma \cos \theta - V g \rho \qquad [N] \qquad (8)$$

wobei F die gemessene Nettokraft, und im ersten Term auf der rechten Seite der Gleichung $P$ der Umfang (Perimeter) der Probe, $\gamma$ die Oberflächenspannung des Wassers und $\theta$ den dynamischen Kontaktwinkel (Vorrückwinkel $\theta_V$ oder Rückzugswinkel $\theta_R$) darstellen. Im zweiten Term bedeuten $V$ das Volumen der verdrängten Flüssigkeit, g die Gravitation und $\rho$ die Dichte der Flüssigkeit. Der zweite Term, der den Auftrieb der Probe in der Flüssigkeit angibt, kann eliminiert werden durch Extrapolation auf die Eintauchtiefe Null und führt zur vereinfachten Form der Wilhelmy-Gleichung:

$$\cos \theta = F / (P \times \gamma) \qquad (9)$$

Wenn $P$ der Einheit von 1 cm (z.B. Plättchen von 10 x 5 x 1 mm) gleichgesetzt wird, erhält man die Konstante $1/(P \cdot \gamma) = K_\theta = 1.39 \cdot 10^3\ N^{-1}$ und somit die Gleichung:

$$\cos \theta = K_\theta \cdot F \qquad (9a)$$

Werden Kontaktwinkel nach Gleichung 9 ohne Extrapolation auf Null berechnet, so werden sie als "virtuelle" Kontaktwinkel bezeichnet. Die Verwendung von virtuellen Kontaktwinkeln wird unten in **Fig. 7** gezeigt.

Praktisch gesehen, ist die Gültigkeit der Gleichung 9 für eine vollkommen glatte Oberfläche durch zwei verbotene Kontaktwinkel eingeschränkt: (i) $\theta \ngtr 119°$ and (ii) $\theta \nless 0°$. Für den ersten Fall auf der hydrophoben Seite ist im Stand der Technik bekannt, dass Kontaktwinkel aus physikalischen Gründen den Wert $\theta = 119°$ nicht überschreiten können. Im zweiten Fall ($\theta \nless 0°$) auf der hydrophilen Seite kann der Kontaktwinkel aus dem mathematischen Grund, dass cos $\theta > 1$ nicht definiert ist, nicht kleiner als Null werden. Seitens des Erfinders wurde nun gefunden, dass diese letztere Barriere nach klassischem mathematischen Verständnis überwunden werden kann, wenn die Kontaktwinkel in den imaginären Zahlenbereich ai erweitert werden und so die Oberflächeneigenschaften der Implantate bewertet werden können.

Für die Messungen wurden seitens des Erfinders Metallplättchen aus einer mit Reintitan beschichteten Titanlegierung (Ti-6Al-4V) (sog. Titan-Plasma-Spray Verfahren, TPS) mit einer Rauhigkeit von Ra = 30μm und einer mikroskopischen Rauhigkeit von $r_m$ = 20 verwendet. Dynamische Kontaktwinkel $\theta_V$ (Vorrückwinkel) und $\theta_R$ (Rückzugswinkel) wurden mit ultrareinem Wasser nach der Methode von Wilhelmy bestimmt (Tensiometer DCAT 11, Dataphysics, Filderstadt, Germany). Eintauch- und Auftauchgeschwindigkeit betrugen 1 mm/min (17 μm/s) so dass die gemessenen Kontaktwinkel unabhängig von der Tauchgeschwindigkeit sind. Die apparenten statischen Kontaktwinkel $\theta_S$' (sitzende Tropfenmethode; 3-5 μl ultrareines Wasser) wurden grafisch ausgewertet. Aus den gemessenen KraftWerten wurden durch den Erfinder die imaginären Kontaktwinkel berechnet.

Die festgestellte "extreme Hydrophilie ($\theta$ > 0i) ("Hyperhydrophilie") - hier in Abwesenheit von Hysterese auf einer mikrorauhen Oberfläche" nach einer nasschemischen Behandlung (Säureätzung) - bezeichnet der Erfinder hier mit dem Begriff "Inverser Lotus Effekt". Dieser Begriff findet auch Anwendung in der Beschreibung hyperhydrophiler Oberflächen, die über ein sog. "chemical switching" aus einer den "Lotus Effect" zeigenden Oberfläche entstanden. So wird eine hyperhydrophobe Oberfläche (**Fig. 5**) durch Behandlung mit Chromschwefelsäure in eine hyperhydrophile Oberfläche "umgeschaltet", wobei letztere nach bisherigen analytischen Methoden eine extreme Spreitung von Wasser $(\theta_S^{H_2O} = 0°)$ und dynamische Kontaktwinkel von $\theta_V/\theta_R$ = 0°/0°) (**Fig. 6**) aufweist. Da n-Hexan und Mineralöl auf diesen Oberflächen spreiten $(\theta_S^{H_2O} / \theta_S^{Oil} / \theta_S^{n-Hexane} \sim 0°/0°/0°)$, werden sie auch als superamphiphil bezeichnet. Die "Rückumwandlung" vom hyper-hydrophilen in den hydrophoben Zustand erfolgt spontan langsam an der Luft, sofern die Oberfläche nicht konserviert wird.

Ebenso ist die Erfindung auf ein Verfahren gerichtet, dass die Behandlung zur Erzeugung einer Nanostruktur den Schritt einer nasschemischen Behandlung der mikrostrukturierten Oberfläche umfasst, wobei eine hydrophobe oder

schwach hydrophile Oberfläche in eine ultra- oder hyperhydrophile Oberfläche umgewandelt wird, wobei zumindest einer der beiden dynamischen Kontaktwinkel ($\theta_V$ und $\theta_R$) im Bereich $\frac{\Delta F}{P \cdot \gamma} = 0.980$ bis $2.15$ und bevorzugt im hyperhydrophilen Bereich $\frac{\Delta F}{P \cdot \gamma} > 1.0$ bis $1.0619$ ($\theta_a^i > 0° - 0.35i$ rad) liegt.

Die Beobachtungen des Erfinders zu dem sequentiellen Vorkommen zweier verschiedener Lotus-Effekte auf ein und derselben Oberfläche nach "chemical switching" deutet darauf hin, dass es eine Verbindung zwischen diesen beiden Effekten gibt, die jedoch noch unklar ist. Im hydrophoben Fall ist der Einfluß der Rauhigkeit auf den dynamischen Kontaktwinkel ($\theta_V'/\theta_R'$ = 98.8°/36.7°) über eine Erhöhung auf $\theta_S^{H_2O} \sim 145°$ (statische Methode) durch die heterogene Benetzung klar zu erkennen. Auf der hydrophilen Seite fehlt jedoch ein analoger Effekt der Oberflächenrauhigkeit auf einen Kontaktwinkel von Null. Die Messungen des Erfinders ergaben, dass alle Kontaktwinkel, die im Bereich von cos $\theta > 1$ lagen, als Kontaktwinkel mit dem Wert Null ausgegeben wurden. Eine erfindungsgemäße Auswertung der Rohdaten der **Fig. 6** zeigt nun, dass 17% der Meßpunkte in **Fig. 6** nicht definierte Kontaktwinkel " mit cos $\theta > 1$ ergeben. Diese Beobachtung ist im Profil von **Fig. 6** durch eine Demarkationslinie dargestellt, die den definierten vom undefinierten Bereich trennt. Seitens des Erfinders wurde nun ein Weg gefunden, um die Daten der Wilhelmy-Messungen aus dem undefinierten Zustand in einen definierten zu bringen.

**Tabelle 3**

| Gültigkeits-bereich | Kontinuierlicher Bereich der imaginären und reellen Kontaktwinkel | | **$K_\theta \cdot F$** |
|---|---|---|---|
| | **arccos ($K_\theta \cdot F$), rad** | **$\theta^a_{ai}$, Grad** | |
| | | | |
| | 1.40i | 80.21i | 2.1509 |
| | 1.23i | 70.47i | 1.8568 |
| | 1.05i | 60.16i | 1.6038 |
| | 0.87i | 49.85i | 1.4029 |
| | 0.71i | 40.68i | 1.2628 |
| | 0.53i | 30.37i | 1.1438 |
| Zentralbereich des inversen Lotus-Effekts | 0.35i | 20.05i | 1.0619 |
| | 0.18i | 10.31i | 1.0162 |
| | 0.04i | 2.29i | 1.0008 |
| | 0.00 | 0i | +1.0 |
| | Hyperhydrophilie | ⇑ $\cos \theta \geq 1$) | |
| | Ultrahydrophilie | $\cos \theta \leq 1$) ⇓ | |
| | 0.00 | 0 | +1.0 |
| | 0.04 | 2.29 | 0.9992 |
| | 0.18 | 10.31 | 0.9838 |
| | 0.35 | 20.05 | 0.9397 |
| | 0.53 | 30.37 | 0.8628 |
| | 0.71 | 40.68 | 0.7584 |
| | 0.87 | 49.85 | 0.6448 |
| | 1.05 | 60.16 | 0.4976 |
| | 1.23 | 70.47 | 0.3342 |
| | 1.40 | 80.21 | 0.1699 |
| | 1.57 | 90.00 | 0 |
| | 3.14 | 180.00 | -1.0 |
| | $K_\theta$ | | |

(i) Tabelle 3 zeigt klassische und neue imaginäre Kontaktwinkel $\theta^a_{ai}$ in Radiant und Grad, die nach den ($K_\theta \cdot F$) Werten berechnet wurden. Dabei verhalten sich imaginäre und reelle Kontaktwinkelreihen wie Spiegelbilder zu Null. Der "Inverse Lotus Effekt" reicht von 0.18 rad (~10°) im reellen bis 1.4i rad (~80°) im imaginären Zahlenraum bevorzugt von 0.18 rad bis 0.35 rad [~20°). Dabei kann einer der dynamischen Kontaktwinkel (z.B. $\theta_A$) klassisch und der zweite (z.B. $\theta_{ai,R}$) imaginär sein, was als hybrides Kontaktwinkelpaar bezeichnet

wird. Anderseits können auch beide dynamischen Kontaktwinkel ($\theta_{ai,V} / \theta_{ai,R}$) imaginär sind (reines imaginäres Kontaktwinkelpaar). Multiplikation vom Radianten-Wert mit 180/π führt zum Kontaktwinkel in Grad: 57.3 x 0.4i [rad] = 22.9i°. Für $K_\theta \cdot F > 1{,}0$ ergibt sich ein imaginärer Kontaktwinkel von >0,0i rad respektive >0,0i°. Dies wird als untere Grenze für imaginäre Kontaktwinkel.definiert

Diese Befunde ergeben die Erweiterung der Wilhelmy-Gleichung in den imaginären Zahlenraum:

$$\cos \theta_{ai}^{a} = F / (P \times \gamma) \qquad (10)$$

Der allgemeine Ausdruck $\theta_{ai}^{a}$ bezeichnet alle Kontaktwinkel im reellen Raum (hochgestelltes a) für die Randbedingung ($K_\theta \cdot F$) < 1 und alle Kontaktwinkel im imaginären Raum (tiefgestelltes ai) für die Randbedingung ($K_\theta \cdot F$) > 1 (siehe Tabelle 3).

Mit Hilfe der Gleichung 10 können nun definierte Kontaktwinkel für alle Kraftmessungen im Bereich ($K_\theta \cdot F$) = -1.0 bis +2.15 ausgehend vom reellen Zahlensystem des cos (180°) bis zum imaginären System von cos (80i°) (siehe Tabelle 3) angegeben werden. Größere imaginäre Kontaktwinkel bis zu 180i° sind nach Vermutungen des Erfinders auf rauhen Oberflächen denkbar.

Der Einsatz imaginärer Kontaktwinkel zur Bestimmung einer hoch-benetzbaren TPS-Oberfläche ist in **Fig. 7** gezeigt. Es wurden 45 repräsentative Werte der Rohdaten oberhalb der Demarkationslinie bei 0.102 g in **Fig. 6** ausgewählt und deren Kraftwerte ($K_\theta \cdot F$; im bereich 1.00 bis 1.07) in virtuelle imaginäre Kontaktwinkel (θai) umgerechnet und als Funktion der Eintauchtiefe aufgetragen. Die Extrapolation des linearen Anteils der Kurven auf die Position Null der Eintauchtiefe, ergab die apparenten imaginären Vorrück- und Rückzugswinkel ($\theta_{ai,V} / \theta_{ai,R}$) = 0.36i°/0.37i°). Die so ermittelten imaginären Kontaktwinkel sind eine komplexe Funktion der vier Benetzungsgrößen Cohäsion, Adhäsion, Spreitung und Immersion. Sie enthalten die Informationen dieser Benetzungsgrößen einschließlich der Wasseraufnahme und sind daher für die Benetzung der dargestellten rauhen Oberfläche charakteristisch.

Somit ist es anhand der Erkenntnisse des Erfinders möglich, die Eigenschaften von solchen hydrophilen Oberflächen, für die bisher solche Bestimmungen wie bei hyperhydrophilen Oberflächen nicht möglich war, zu bestimmen und ihre Eignung für nachfolgende Behandlungen einschließlich Beschichtungen bewerten zu können.

## Patentansprüche

1. Implantat mit einer mikrostrukturierten hyperhydrophilen Oberfläche mit Erhebungen und Vertiefungen, wobei der Abstand zwischen den Erhebungen im statistischen Mittel im Bereich von 1 bis 100 μm und die Profilhöhe der Erhebungen und Vertiefungen im statistischen Mittel (Ra-Wert) im Bereich von 1 bis 80 μm liegen, wobei das Implantat einen mikroskopischen Rauhigkeitsfaktor $r_M$ im Bereich zwischen 2 und 50 besitzt, wobei zumindest einer der beiden dynamischen Kontaktwinkel ($\theta_V$ und $\theta_R$) im hyperhydrophilen Bereich mit $\frac{\Delta F}{P \cdot \gamma} > 1{,}0$ bis 2,15 ($\theta_{ai}$ > 0,0i° - 80i° ), besonders mit $\frac{\Delta F}{P \cdot \gamma} > 1{,}0$ bis 1,0619 ($\theta_{ai}$ > 0,0i° - 20i°) liegt.

2. Implantat nach Anspruch 1, wobei die erste Mikrostruktur mit den ersten Erhebungen und Vertiefungen von einer zweiten Mikrostruktur mit zweiten Erhebungen und Vertiefungen überlagert ist, wobei der Abstand zwischen den zweiten Erhebungen im statistischen Mittel im Bereich von 0,1 bis 10 μm liegt und die Höhe der zweiten Erhebungen und Vertiefungen im statistischen Mittel (Ra-Wert) im Bereich von 0,1 bis 10 μm liegt.

3. Implantat nach Anspruch 1 oder 2, wobei die hyperhydrophile Oberfläche unregelmäßig oder zumindest in Teilbereichen regelmäßig mikrostrukturiert ist.

**4.** Implantat nach Anspruch 1, 2 oder 3, wobei die mikrostrukturierten hyperhydrophilen Oberflächen mit einer Nanostruktur überlagert sind, die vorzugsweise auf nasschemischem Wege durch Säureätzung erzeugt wurde.

**5.** Verfahren zur Herstellung eines Implantates mit einer regelmäßig mikrostrukturierten Oberfläche mit Erhebungen und Vertiefungen nach Anspruch 1, das die Schritte aufweist:

a. Bereitstellung eines Pulvers oder einer Pulvermischung aus einem sinterbaren Materialpulver auf einem Rohling;
b. Aufbringen einer Schicht des Materialpulvers auf der Oberfläche des Rohlings;
c. Beaufschlagen der Schicht des Materialpulvers mit energiereicher Strahlung in einem Muster, das aus einer in einen STL-Datensatz überführten periodischen Funktion darstellbar ist, so dass Materialpulver unter Ausbildung mindestens eines Teilbereiches des Musters auf zumindest einem Teilbereich der Oberfläche des Rohlings gesintert wird.

**6.** Verfahren nach Anspruch 5, bei dem der Rohling aus Vollmaterial oder schichtweise über ein Sinterverfahren aus einem sinterbaren Materialpulver hergestellt ist.

**7.** Verfahren nach einem der Ansprüche 5 oder 6, bei dem der in c) erhaltene Rohling mit einer regelmäßig mikrostrukturierten Oberfläche einer Behandlung zur Erzeugung einer zweiten regelmäßigen Mikrostruktur unter Verwendung einer aus einer in einen STL-Datensatz überführten periodischen Funktion und/oder einer nasschemischen Behandlung zur Erzeugung einer Nanostruktur unterzogen wird.

**8.** Verfahren zur Herstellung eines Implantates mit einer regelmäßig mikrostrukturierten Oberfläche nach Anspruch 1, das die Schritte aufweist:

a. Bereitstellen eines Rohlings;
b. Beaufschlagen des Rohlings mit energiereicher Strahlung zumindest teilweise in einem Muster, das aus einer in einen STL-Datensatz überführten periodischen Funktion darstellbar ist, so dass unter Ausbildung mindestens eines Teilbereiches des Musters auf zumindest einem Teilbereich der Oberfläche der Rohling abgetragen wird.

**9.** Verfahren nach Anspruch 8, bei dem der in b) erhaltene Rohling mit einer regelmäßig mikrostrukturierten Oberfläche einer Behandlung zur Erzeugung einer zweiten regelmäßigen Mikrostruktur unter Verwendung einer aus einer in einen STL-Datensatz überführten periodischen Funktion und/oder einer nasschemischen Behandlung zur Erzeugung einer Nanostruktur unterzogen wird.

**10.** Verfahren nach einem der Ansprüche 7 oder 9, wobei die Behandlung zur Erzeugung einer Nanostruktur den Schritt einer nasschemischen Behandlung der mikrostrukturierten Oberfläche umfasst, wobei eine hydrophobe oder schwach hydrophile Oberfläche in eine ultra- oder hyperhydrophile Oberfläche umgewandelt wird, wobei zumindest einer der beiden dynamischen Kontaktwinkel ($\theta_V$ und $\theta_R$) im hyperhydrophilen Bereich mit $\frac{\Delta F}{P \cdot \gamma} > 1{,}0$ bis $2{,}15$ ($\theta_{ai} > 0{,}0i° - 80i°$), besonders mit $\frac{\Delta F}{P \cdot \gamma} > 1{,}0$ bis $1{,}0619$ ($\theta_{ai} > 0{,}0i° - 20i°$) liegt.

**11.** Verfahren nach Anspruch 10, das den zusätzlichen Schritt umfasst, dass die erhaltene Oberfläche mit Hilfe einer Lösung nicht-flüchtiger Substanzen wie Salzen, organischen Lösungsmitteln, die nicht mit der Oberfläche wechselwirken, oder einer salzhaltigen Exsikkationsschicht zum Schutz der Oberfläche des Substrates gegenüber einer Benetzungsabnahme mit Verlust der Hyperhydrophilie durch Alterung oder durch Sterilisierungsverfahren (z.B. Gammasterilisierung) geschützt, stabilisiert und langzeitlagerfähig gemacht wird.

**12.** Verfahren nach einem der Ansprüche 5 bis 11, bei dem die eine in einen STL-Datensatz überführte periodische Funktion eine trigonometrische Funktion $A_R(x)$ ist, die aus der Gruppe ausgewählt wird, die besteht aus:

$$A_R(x) = (\sin(x) \; ,$$

$$A_R(x) = \frac{4a}{\pi}(\sin(x) + \frac{1}{3}\sin[3x] + \frac{1}{5}\sin(5x) + \frac{1}{7}\sin(7x) + \frac{1}{9}\sin(9x) + .....) \, ,$$

$$A_R(x) = \frac{4a}{\pi}\left(\sin(x) - \left(\frac{1}{3}\right)^2 \sin(3x) + \left(\frac{1}{5}\right)^2 \sin(5x) - \left(\frac{1}{7}\right)^2 \sin(7x) + \left(\frac{1}{9}\right)^2 \sin(9x) + .....\right) ,$$

$$A_R(x) = \frac{2a}{\pi}(\sin(x) - \frac{1}{2}\sin[2x] + \frac{1}{3}\sin(3x) - \frac{1}{4}\sin(4x) + \frac{1}{5}\sin(5x) + .....) \, ,$$

und Ableitungen davon.

**13.** Verfahren nach einem der Ansprüche 5 bis 12, bei dem der Rauhigkeitsparameter Ra im Bereich zwischen 1 und 80 µm, bevorzugt zwischen 5 und 60 µm und besonders bevorzugt zwischen 10 und 60 µm liegt.

**14.** Verfahren nach einem der Ansprüche 5 bis 12, bei dem der Periodizitätswert n(λ/2) im Bereich zwischen 1 und 100 µm, bevorzugt zwischen 10 und 60 µm und der mikroskopische Rauhigkeitsfaktor $r_M$ im Bereich zwischen 2 und 50 liegt.

**Claims**

**1.** An implant with a microstructured hyperhydrophilic surface with peaks and valleys, wherein the statistical mean distance between the peaks is in the range of 1 to 100 pm, and the statistical mean profile height of the peaks and valleys (Ra value) is in the range of 1 to 80 pm, wherein the implant has a microscopic roughness factor $r_M$ in the range between 2 and 50, wherein at least one of the two dynamic contact angles ($\theta_V$ and $\theta_R$) is in the hyperhydrophilic range with $\frac{\Delta F}{P \cdot \gamma}$ > 1.0 to 2.15 ($\theta_{ai}$ > 0.0i° - 80i°), in particular with $\frac{\Delta F}{P \cdot \gamma}$ > 1.0 to 1.0619 ($\theta_{ai}$ > 0.0i° - 20i°).

**2.** The implant according to claim 1, wherein the first microstructure with the first peaks and valleys is superimposed by a second microstructure with second peaks and valleys, wherein the statistical mean distance between the second peaks is in the range of 0.1 to 10 pm, and the statistical mean height of the second peaks and valleys (Ra value) is in the range of 0.1 to 10 pm.

**3.** The implant according to claim 1 or 2, wherein the hyperhydrophilic surface is microstructured irregularly, or regularly at least in sections.

**4.** The implant according to claim 1, 2 or 3, wherein the microstructured hyperhydrophilic surfaces are superimposed with a nanostructure, which was preferably generated by wet chemical acid etching.

**5.** A method for producing an implant with a regular microstructured surface with peaks and valleys according to claim 1, comprising the steps of:

a. providing a powder or a powder mixture of a sinterable material powder on a blank;
b. applying a layer of the material powder on the surface of the blank;
c. applying high-energy radiation to the layer of material powder in a pattern which can be represented by a periodic function which has been transformed into an STL data set, so that material powder is sintered to form at least a section of the pattern on at least a section of the surface of the blank.

**6.** The method of claim 5, wherein the blank is produced from solid material, or layerwise from a sinterable material powder by means of a sintering process.

**7.** The method according to any one of claims 5 or 6, wherein the blank obtained in c) having a regularly microstructured surface is subjected to a treatment to generate a second regular microstructure using a periodic function which has been transformed into an STL data set, and/or to a wet chemical treatment to generate a nanostructure.

**8.** A method for producing an implant with a regularly microstructured surface according to claim 1, comprising the steps of:

a. providing a blank;
b. applying high-energy radiation to the blank at least partially in a pattern which can be represented by a periodic function which has been transformed into an STL data set, so that the blank is ablated to form at least a section of the pattern on at least a section of the surface.

**9.** The method according to claim 8, wherein the blank obtained in b) with a regularly microstructured surface is subjected to a treatment to generate a second regular microstructure using a periodic function which has been transformed into an STL data set, and/or to a wet chemical treatment to generate a nanostructure.

**10.** The method according to any one of claims 7 or 9, wherein the treatment to generate a nanostructure comprises the step of a wet-chemical treatment of the microstructured surface, wherein a hydrophobic or weakly hydrophilic surface is converted into an ultra- or hyperhydrophilic surface, wherein at least one of the two dynamic contact angles ($\theta_V$ and $\theta_R$) is in the hyperhydrophilic range with $\frac{\Delta F}{P \cdot \gamma}$ > 1.0 to 2.15 ($\theta_{ai}$ > 0.0i° - 80i°), in particular with $\frac{\Delta F}{P \cdot \gamma}$ > 1.0 to 1.0619 ($\theta_{ai}$ > 0.0i° - 20i°) .

**11.** The method according to claim 10, comprising the additional step of protecting, stabilising or providing long-term storability to the obtained surface using a solution of non-volatile substances such as salts, organic solvents that do not interact with the surface, or a salt-containing desiccant layer to protect the surface of the substrate from a decrease in wetting with loss of hyperhydrophilia by aging or by sterilization methods (e.g., gamma sterilization).

**12.** The method according to any one of claims 5 to 11, wherein the one periodic function which has been transformed into an STL data set, is a trigonometric function $A_R(x)$ selected from the group consisting of:

$$A_R(x) = (\sin(x) ,$$

$$A_R(x) = \frac{4a}{\pi}(\sin(x) + \frac{1}{3}\sin[3x] + \frac{1}{5}\sin(5x) + \frac{1}{7}\sin(7x) + \frac{1}{9}\sin(9x) + .....),$$

$$A_R(x) = \frac{4a}{\pi}(\sin(x) - \left(\frac{1}{3}\right)^2 \sin(3x) + \left(\frac{1}{5}\right)^2 \sin(5x) - \left(\frac{1}{7}\right)^2 \sin(7x) + \left(\frac{1}{9}\right)^2 \sin(9x) + .....),$$

$$A_R(x) = \frac{2a}{\pi}(\sin(x) - \frac{1}{2}\sin[2x] + \frac{1}{3}\sin(3x) - \frac{1}{4}\sin(4x) + \frac{1}{5}\sin(5x) + .....),$$

and derivatives thereof.

**13.** The method according to any one of claims 5 to 12, wherein the roughness parameter Ra is in the range between 1 and 80 pm, preferably between 5 and 60 pm, and particularly preferably between 10 and 60 pm.

**14.** The method according to any one of claims 5 to 12, wherein the periodicity value n(λ/2) is in the range between 1 and 100 pm, preferably between 10 and 60 pm, and the microscopic roughness factor $r_M$ is in the range between 2 and 50.

**Revendications**

1. Implant à surface microstructurée hyperhydrophile comportant des élévations et des creux, dans lequel la distance entre les élévations en moyenne statistique se situe dans la plage de 1 à 100 μm et la hauteur de profil des élévations et des creux en moyenne statistique (valeur Ra) se situe dans la plage de 1 à 80 μm, dans lequel l'implant possède un facteur de rugosité microscopique $r_M$ dans la plage entre 2 et 50, dans lequel au moins l'un des deux angles de contact dynamiques ($\theta_V$ et $\theta_R$) se situe dans la plage hyperhydrophile avec $\frac{\Delta F}{P.\gamma} > 1{,}0$ à $2{,}15$ $(\theta_{ai} > 0{,}0i° - 80i°)$, notamment avec $\frac{\Delta F}{P.\gamma} > 1{,}0$ à $1{,}0619$ $(\theta_{ai} > 0{,}0i° - 20i°)$.

2. Implant selon la revendication 1, dans lequel la première microstructure avec les premiers élévations et creux est recouverte par une deuxième microstructure avec des deuxièmes élévations et creux, dans lequel la distance entre les deuxièmes élévations en moyenne statistique se situe dans la plage de 0,1 à 10 μm et la hauteur des deuxièmes élévations et creux en moyenne statistique (valeur Ra) se situe dans la plage de 0,1 à 10 μm.

3. Implant selon la revendication 1 ou 2, dans lequel la surface hyperhydrophile est microstructurée de façon irrégulière ou régulière au moins dans des zones partielles.

4. Implant selon la revendication 1, 2 ou 3, dans lequel les surfaces microstructurées hyperhydrophiles sont recouvertes d'une nanostructure qui a été générée de préférence par voie chimique à l'état humide par érosion à l'acide.

5. Procédé de fabrication d'un implant avec une surface microstructurée de façon régulière comportant des élévations et des creux selon la revendication 1, qui présente les étapes :

   a. de mise à disposition d'une poudre ou d'un mélange pulvérulent constitué d'une poudre de matériau pouvant être frittée sur une ébauche ;
   b. d'application d'une couche de la poudre de matériau sur la surface de l'ébauche ;
   c. de chargement de la couche de poudre de matériau avec un rayonnement riche en énergie dans un modèle qui peut être représenté à partir d'une fonction périodique convertie en un ensemble de données STL de sorte que de la poudre de matériau soit frittée en formant au moins une zone partielle du modèle sur au moins une zone partielle de la surface de l'ébauche.

6. Procédé selon la revendication 5, dans lequel l'ébauche est fabriquée en matériau plein ou par couche par un procédé de frittage à partir d'une poudre de matériau pouvant être frittée.

7. Procédé selon l'une des revendications 5 ou 6, dans lequel l'ébauche obtenue au point c) avec une surface microstructurée de façon régulière est soumise à un traitement pour générer une deuxième microstructure régulière en utilisant l'un parmi une fonction périodique convertie en un ensemble de données STL et/ou un traitement chimique à l'état humide pour générer une nanostructure.

8. Procédé de fabrication d'un implant avec une surface microstructurée de façon régulière selon la revendication 1, qui présente les étapes :

   a. de mise à disposition d'une ébauche ;
   b. de chargement de l'ébauche avec un rayonnement riche en énergie au moins partiellement dans un modèle qui peut être représenté à partir d'une fonction périodique convertie en un ensemble de données STL de sorte que par la formation d'au moins une zone partielle du modèle sur au moins une zone partielle de la surface, l'ébauche soit enlevée.

9. Procédé selon la revendication 8, dans lequel l'ébauche obtenue au point b) avec une surface microstructurée de façon régulière est soumise à un traitement pour générer une deuxième microstructure régulière en utilisant l'un parmi une fonction périodique convertie en un ensemble de données STL et/ou un traitement chimique à l'état humide pour générer une nanostructure.

**10.** Procédé selon l'une des revendications 7 ou 9, dans lequel le traitement pour générer une nanostructure comprend l'étape d'un traitement chimique à l'état humide de la surface microstructurée, dans lequel une surface hydrophobe ou faiblement hydrophile est transformée en une surface ultra- ou hyperhydrophile, dans lequel au moins l'un des deux angles de contact dynamiques ($\theta_V$ et $\theta_R$) se situe dans la plage hyperhydrophile avec $\frac{\Delta F}{P.\gamma} > 1{,}0$ à $2{,}15$ ($\theta_{ai} > 0{,}0i° - 80i°$), notamment avec $\frac{\Delta F}{P.\gamma} > 1{,}0$ à $1{,}0619$ ($\theta_{ai} > 0{,}0i° - 20i°$).

**11.** Procédé selon la revendication 10, qui comprend l'étape supplémentaire selon laquelle la surface obtenue est protégée, stérilisée et rendue apte à une conservation prolongée à l'aide d'une solution de substances non volatiles telles que des sels, des solvants organiques qui n'interagissent pas avec la surface, ou une couche de dessiccation contenant un sel pour la protection de la surface du substrat contre une élimination du mouillage avec perte de l'hydrophilie par un procédé de vieillissement ou de stérilisation (par exemple stérilisation gamma).

**12.** Procédé selon l'une des revendications 5 à 11, dans lequel une fonction périodique convertie en un ensemble de données STL est une fonction trigonométrique $A_R(X)$ qui est choisie dans le groupe consistant en :

$$A_R(x) = (\sin(x)\ ,$$

$$A_R(x) = \frac{4a}{\pi}(\sin(x) + \frac{1}{3}\sin[3x] + \frac{1}{5}\sin(5x) + \frac{1}{7}\sin(7x) + \frac{1}{9}\sin(9x) + .....),$$

$$A_R(x) = \frac{4a}{\pi}(\sin(x) - \left(\frac{1}{3}\right)^2 \sin(3x) + \left(\frac{1}{5}\right)^2 \sin(5x) - \left(\frac{1}{7}\right)^2 \sin(7x) + \left(\frac{1}{9}\right)^2 \sin(9x) + .....),$$

$$A_R(x) = \frac{2a}{\pi}(\sin(x) - \frac{1}{2}\sin[2x] + \frac{1}{3}\sin(3x) - \frac{1}{4}\sin(4x) + \frac{1}{5}\sin(5x) + .....),$$

et leurs dérivés.

**13.** Procédé selon l'une des revendications 5 à 12, dans lequel le paramètre de rugosité Ra se situe dans la plage entre 1 et 80 µm, de préférence entre 5 et 60 µm et de manière particulièrement préférée entre 10 et 60 µm.

**14.** Procédé selon l'une des revendications 5 à 12, dans lequel la valeur de périodicité n(λ/2) se situe dans la plage entre 1 et 100 µm, de préférence entre 10 et 60 µm et le facteur de rugosité microscopique $r_M$ se situe dans la plage entre 2 et 50.

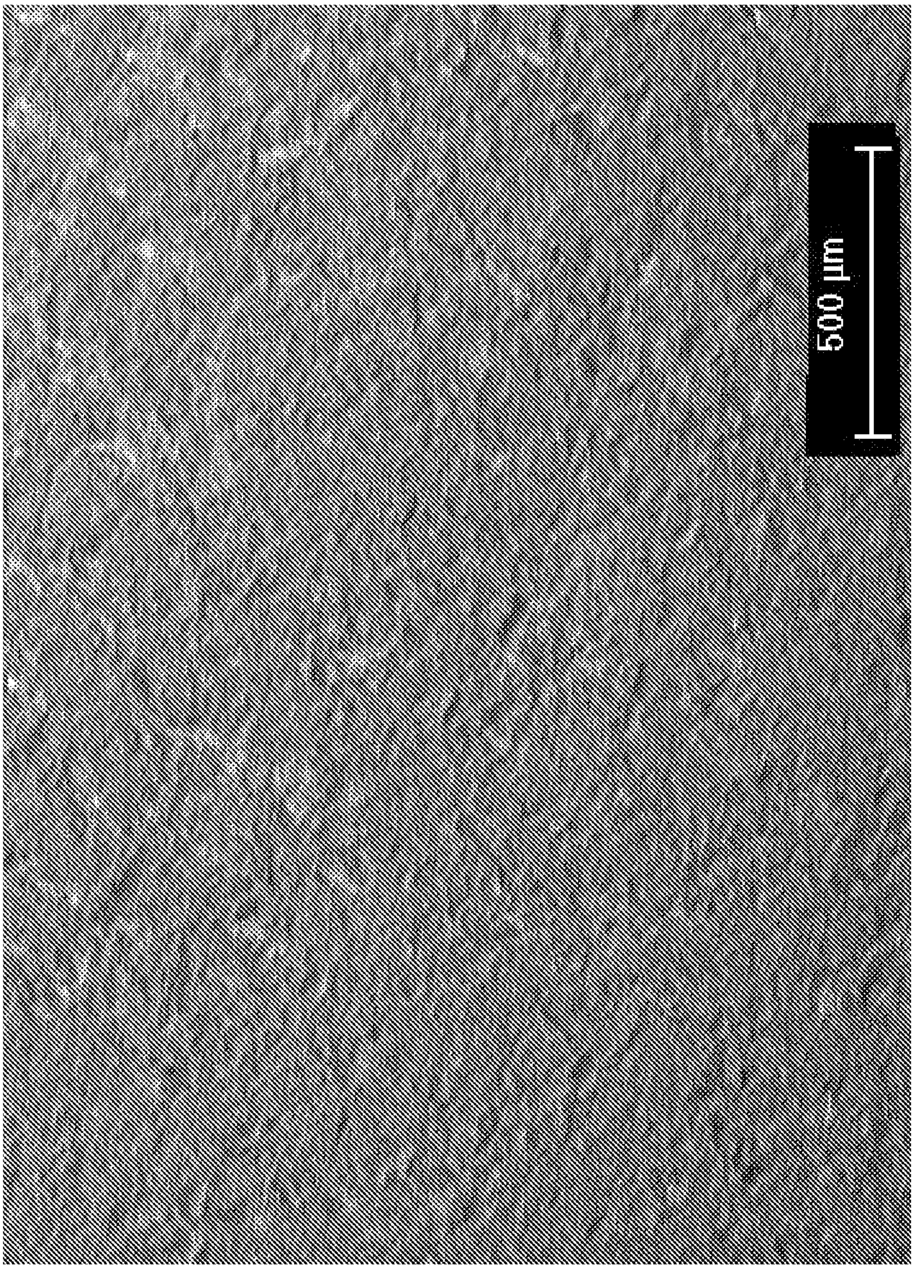


Fig. 1 - A. SLA-Oberfläche 50 x

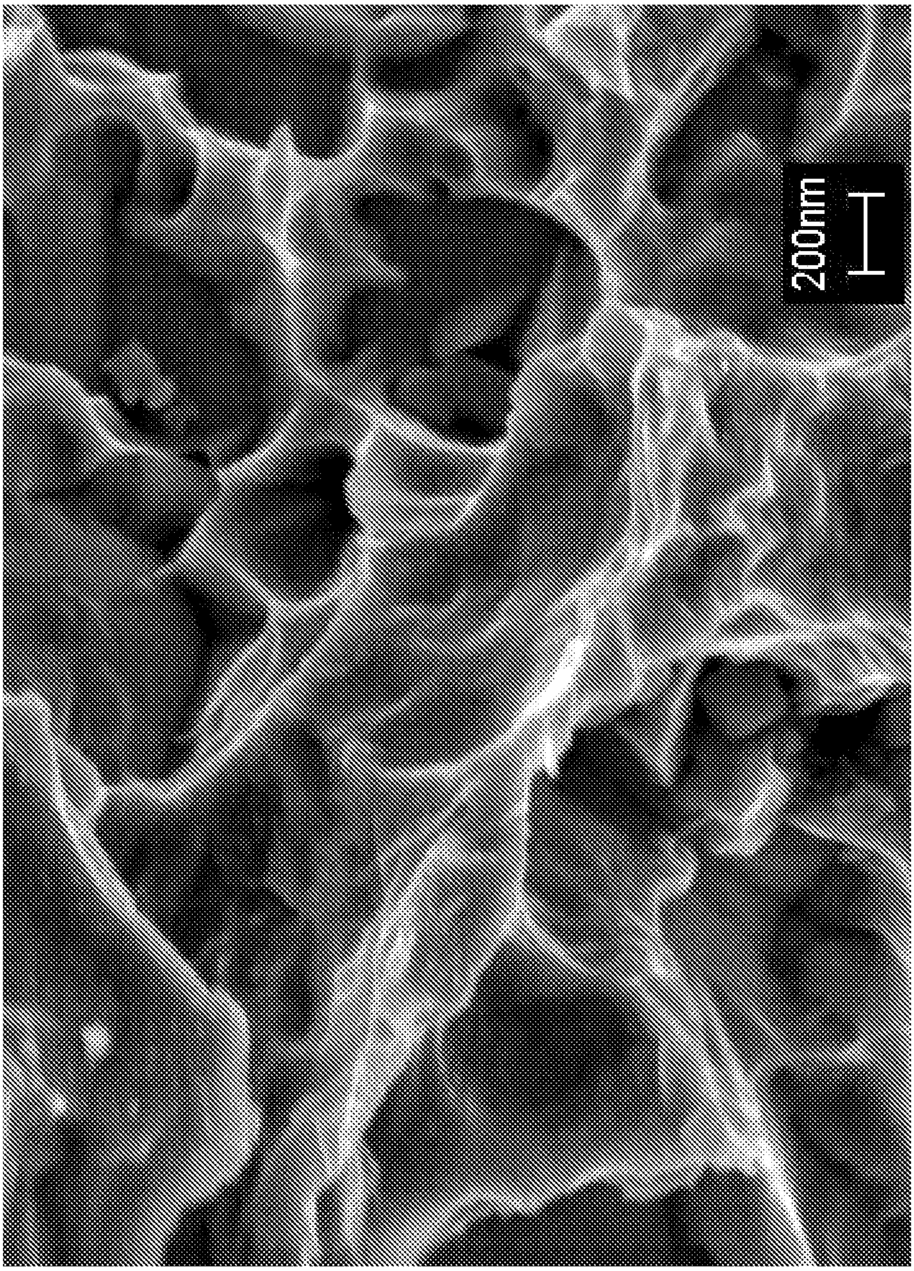


Fig. 1 - B. SLA-Oberfläche 25 000 x

Fig. 1 - C. TPS-Oberfläche 50 x

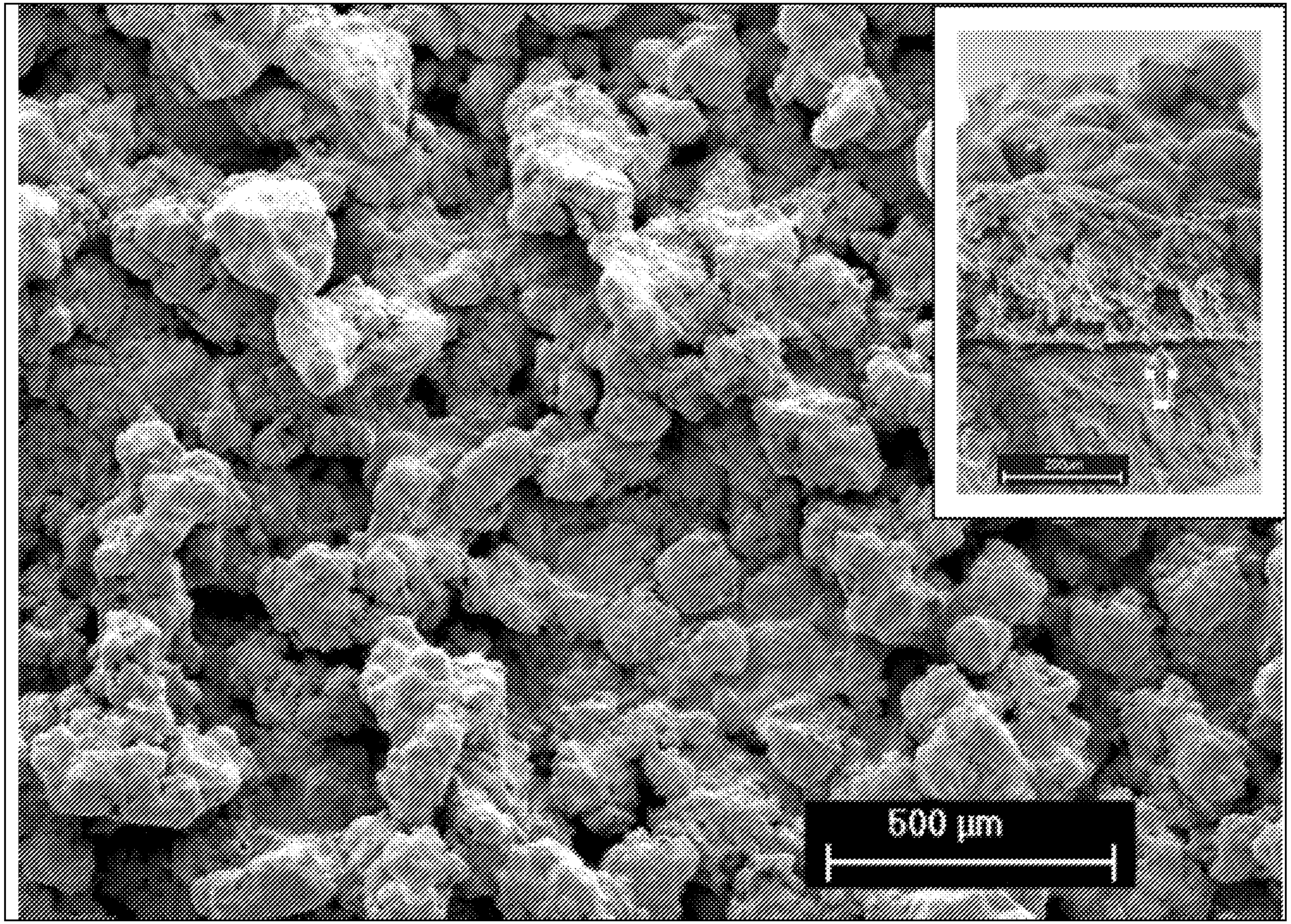

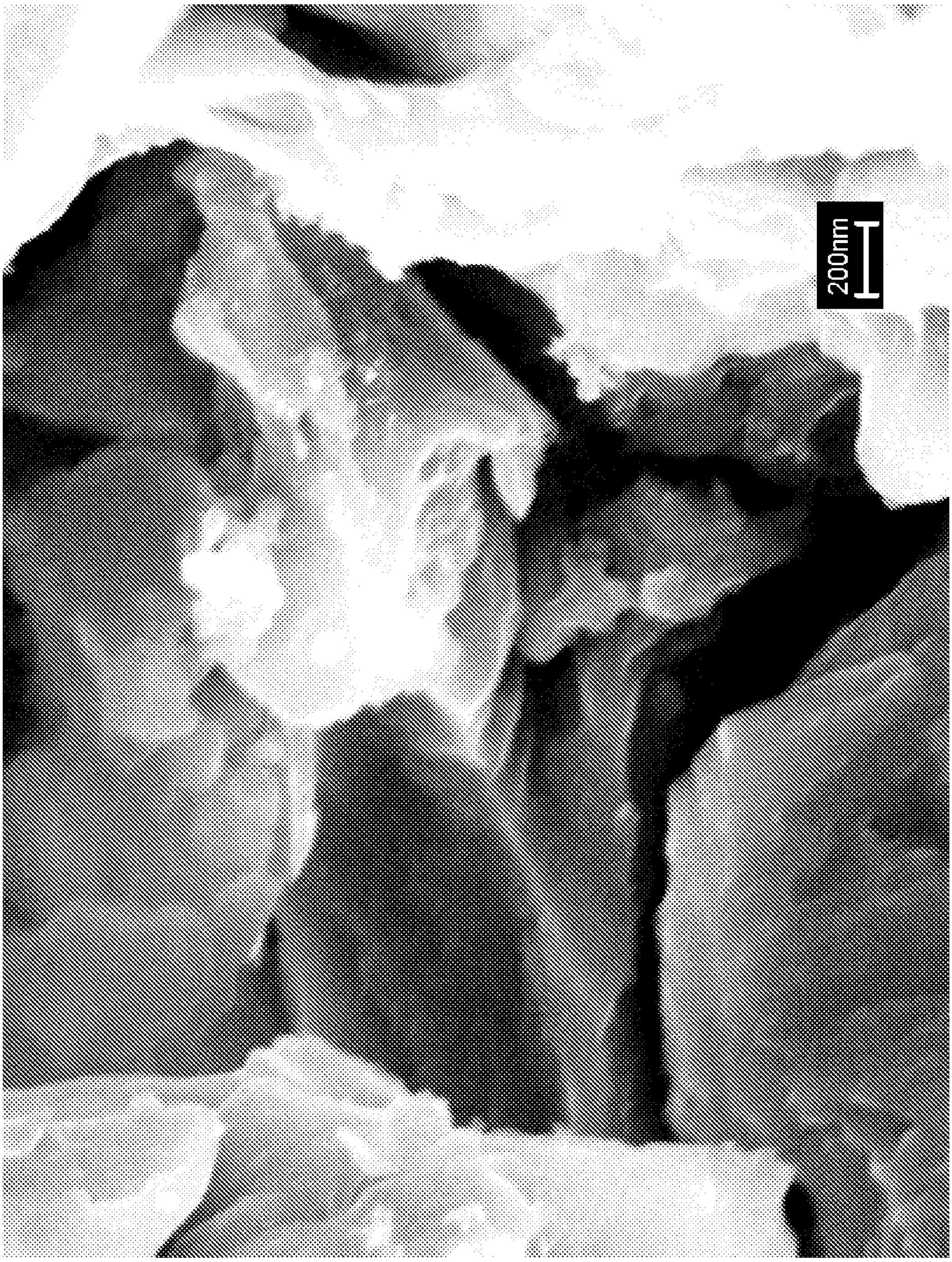


Fig. 1 - D. TPS-Oberfläche 25 000 x

**Fig. 2 - A. Sinusoides Profil (Ra = 50 µm, λ = 32 µm)**

Allgemeine Grundfunktion: $A_R(x) = (\sin(x)$

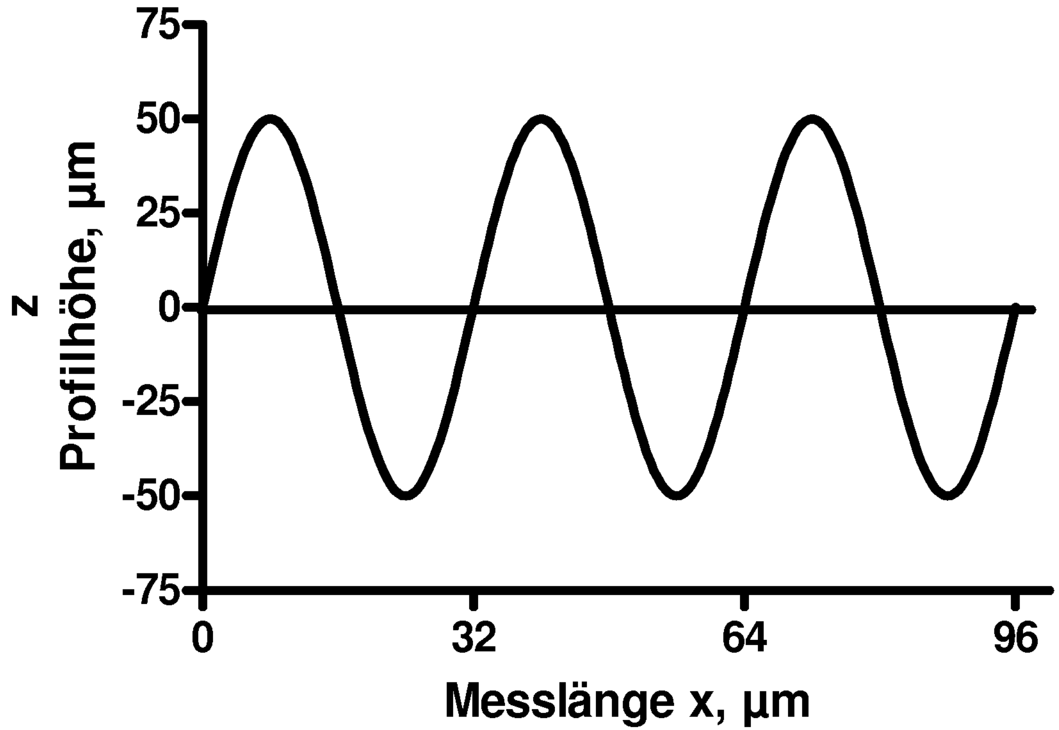


**Definierte Profilfunktion:** $z = A_R(x) = (\sin(\frac{2\pi}{32}x)\square 50$ .

Fig. 2 - B. Rechteckprofil (Ra = 50 µm, λ = 32 µm)

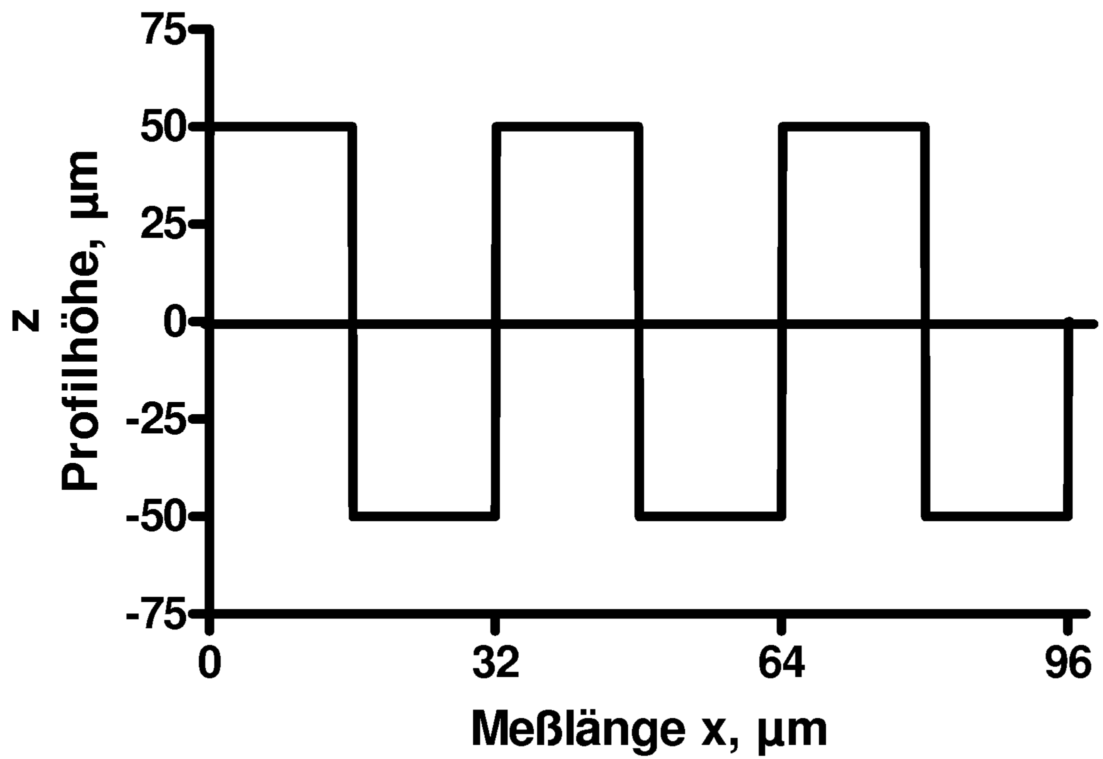


$$A_R(x) = \frac{4a}{\pi}\left(\sin(x) + \frac{1}{3}\sin[3x] + \frac{1}{5}\sin(5x) + \frac{1}{7}\sin(7x) + \frac{1}{9}\sin(9x) + .....\right)$$

Fig. 2 - C. Dreieckprofil (Ra = 50 µm, λ = 32 µm)

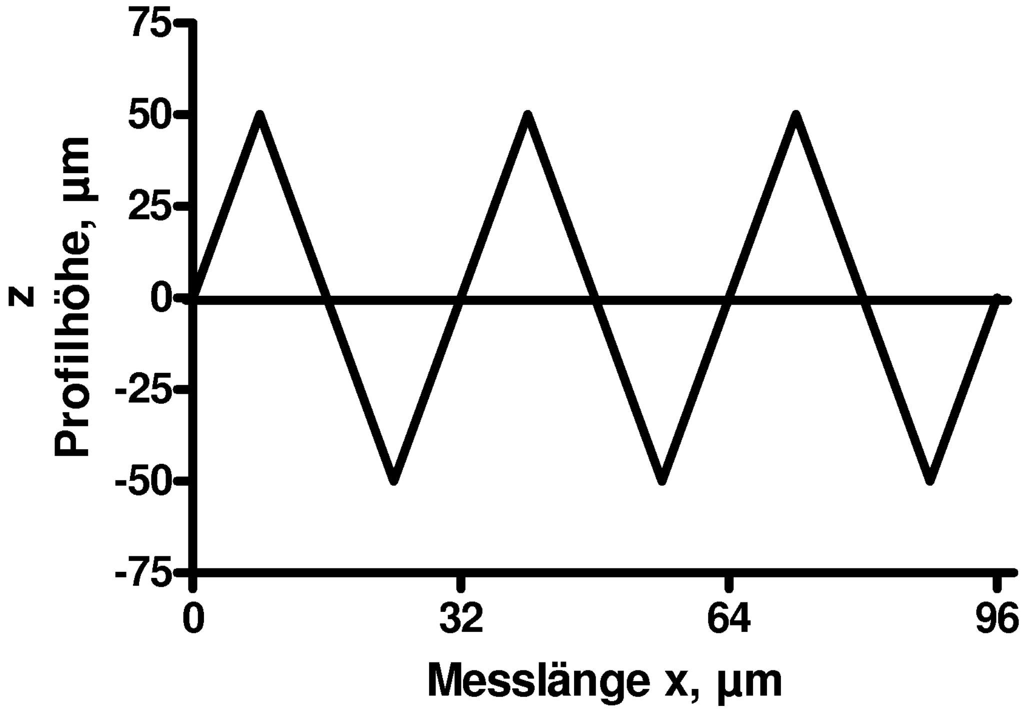


$$A_R(x)=\frac{4a}{\pi}\left(\sin(x)-\left(\frac{1}{3}\right)^2\sin(3x)+\left(\frac{1}{5}\right)^2\sin(5x)-\left(\frac{1}{7}\right)^2\sin(7x)+\left(\frac{1}{9}\right)^2\sin(9x)+\ldots..\right)$$

Fig. 2 - D. Sägezahnprofil (Ra = 25 µm, λ = 32 µm)

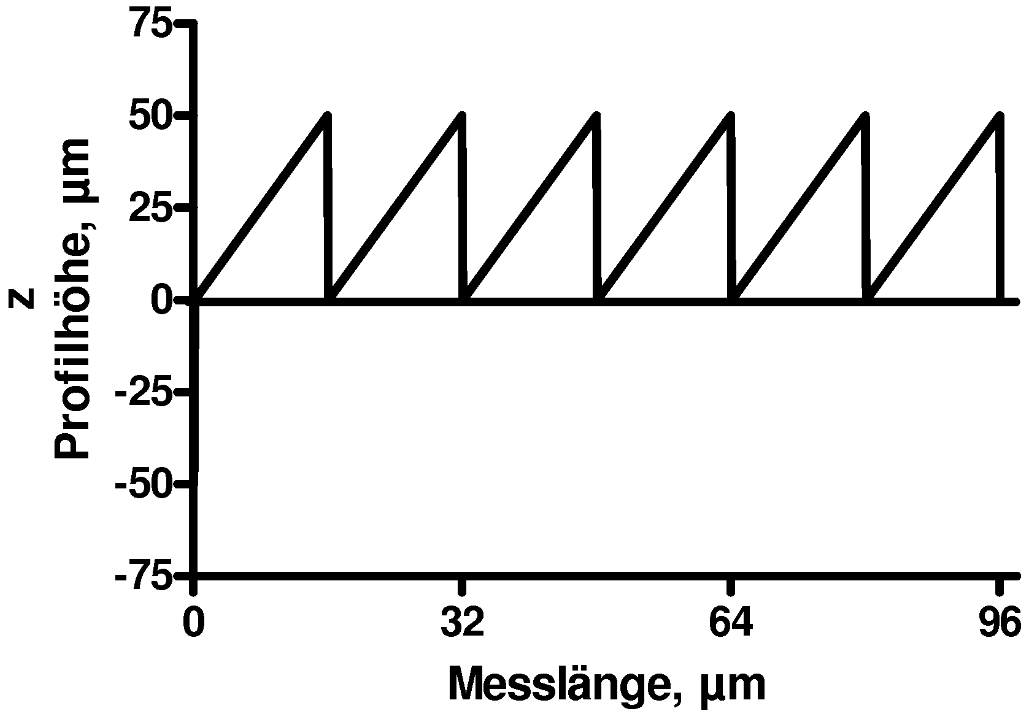


$$A_R(x) = \frac{2a}{\pi}\left(\sin(x) - \frac{1}{2}\sin[2x] + \frac{1}{3}\sin(3x) - \frac{1}{4}\sin(4x) + \frac{1}{5}\sin(5x) + \ldots..\right)$$

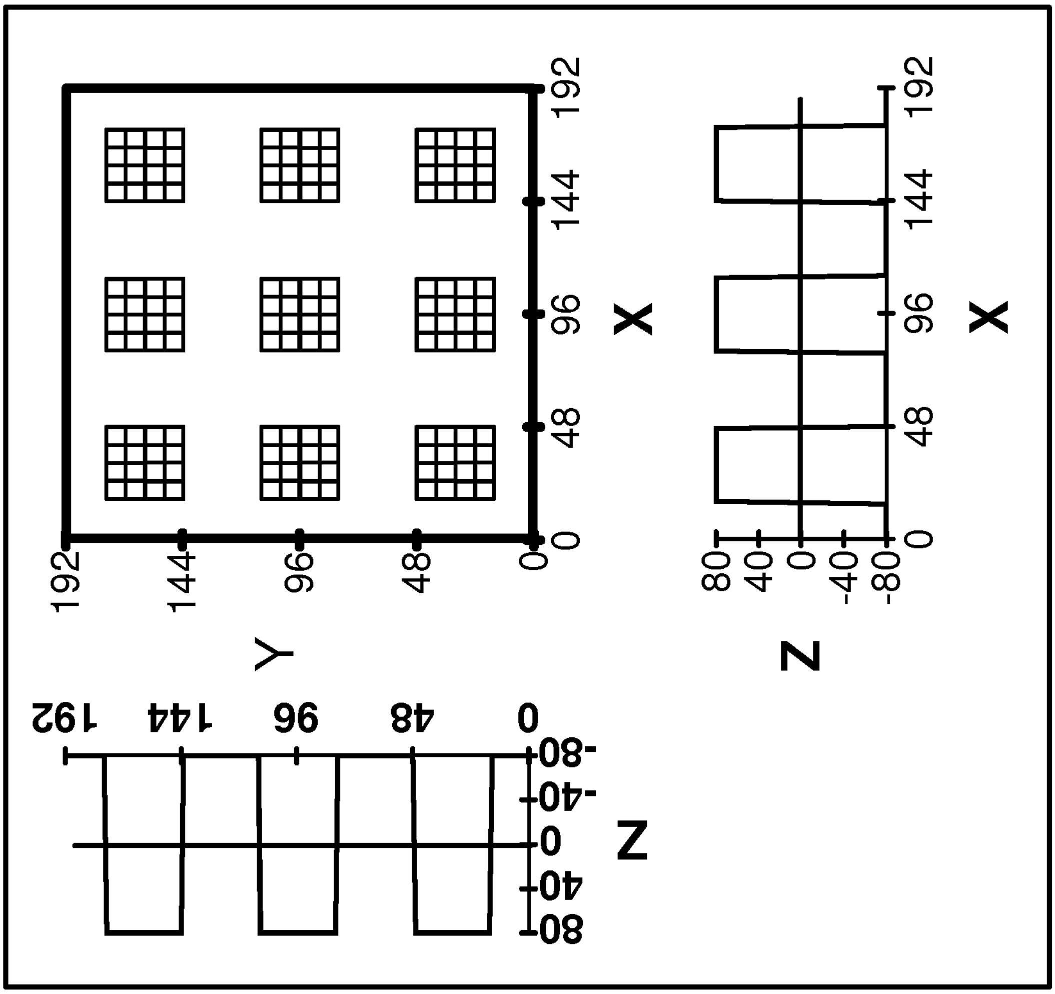


Fig. 3 - A.

Fig. 3 - B.

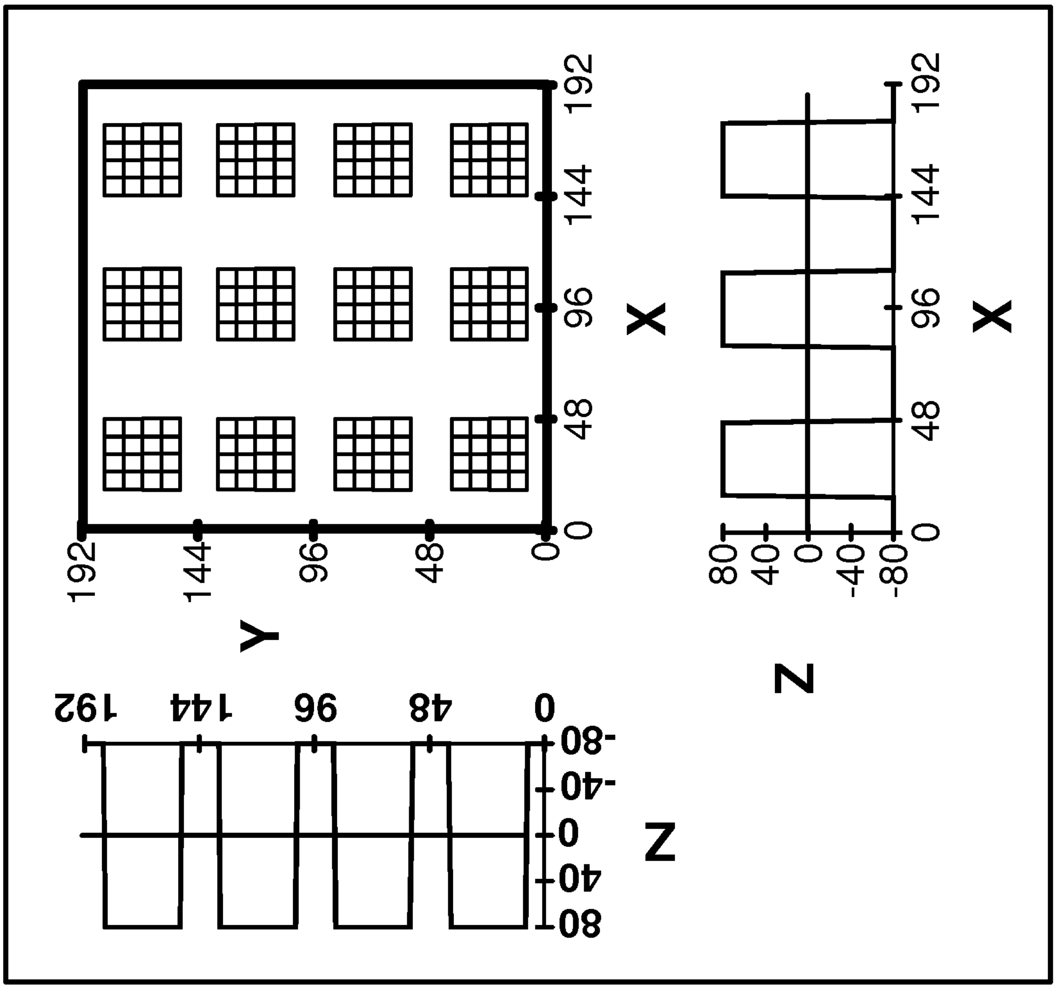

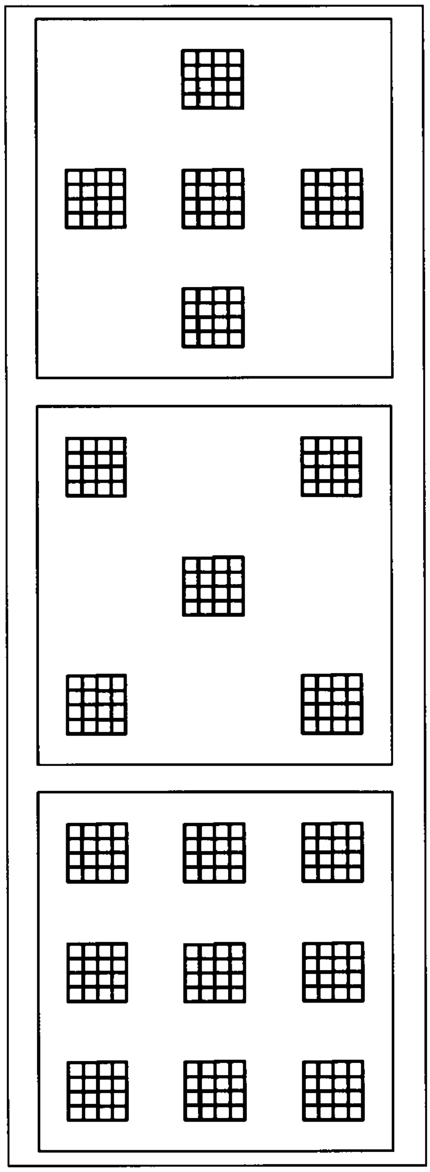

Fig. 3 - C

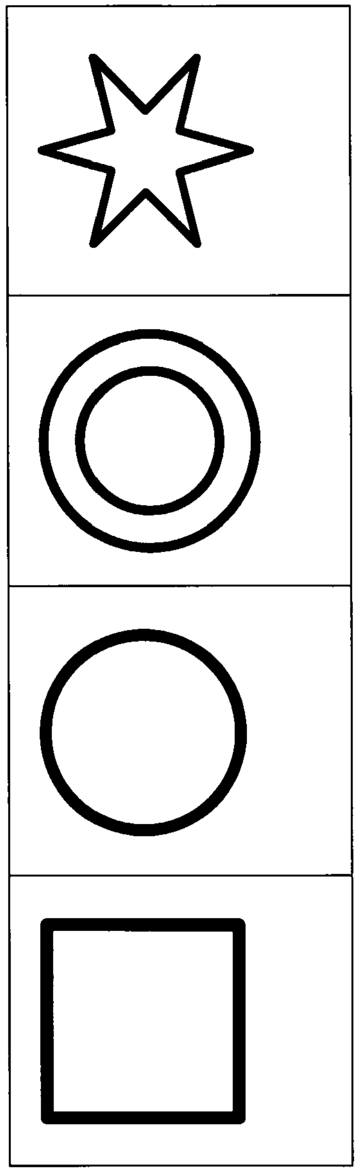

Fig. 3 - D

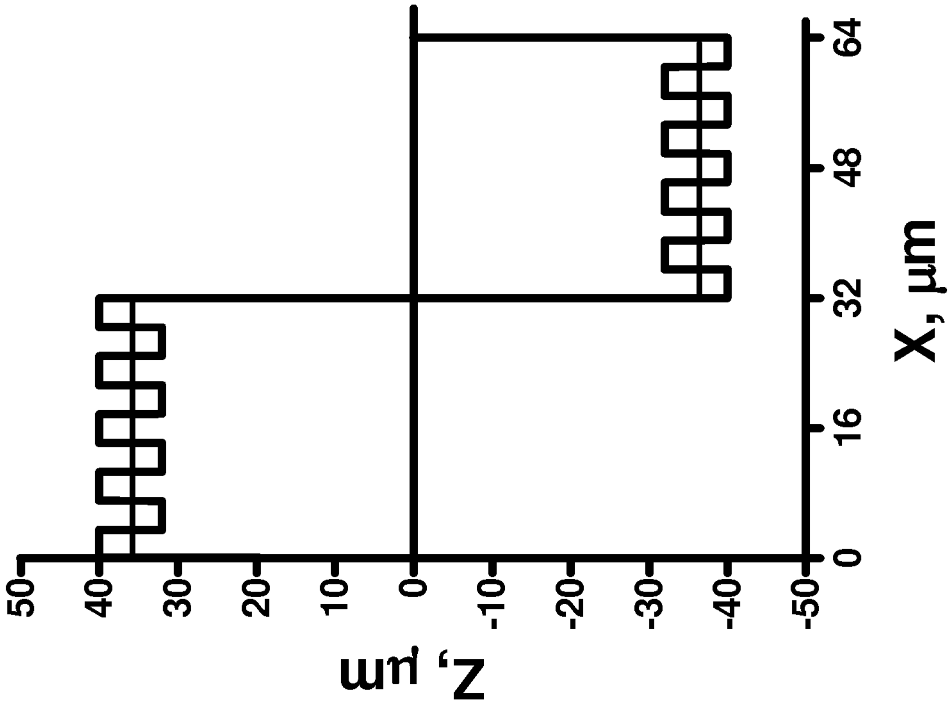


Fig. 4

Fig. 5 - A

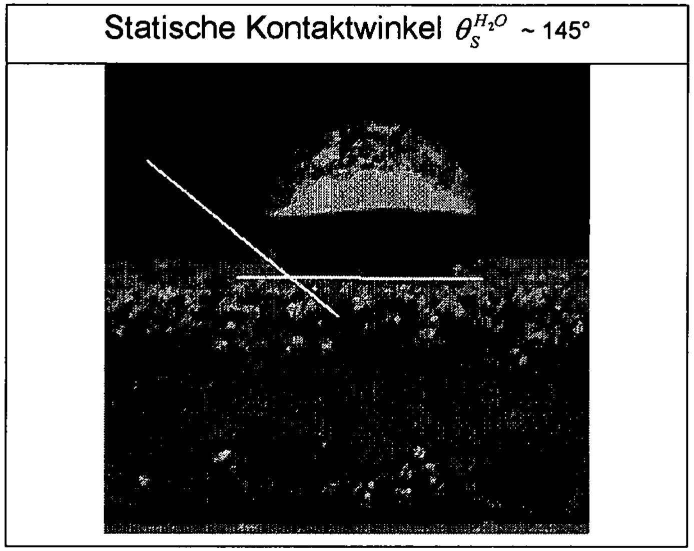
Statische Kontaktwinkel $\theta_S^{H_2O}$ ~ 145°

Fig 5 - B

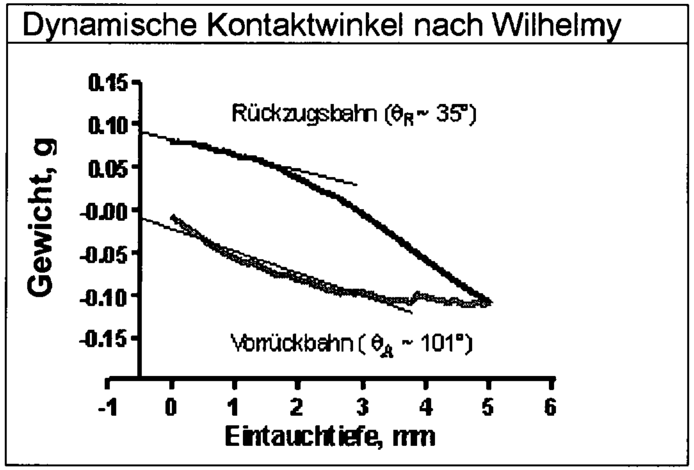
Dynamische Kontaktwinkel nach Wilhelmy
Rückzugsbahn ($\theta_R$ ~ 35°)
Vorrückbahn ($\theta_A$ ~ 101°)
Gewicht, g
0.15
0.10
0.05
-0.00
-0.05
-0.10
-0.15
-1
0
1
2
3
4
5
6
Eintauchtiefe, mm

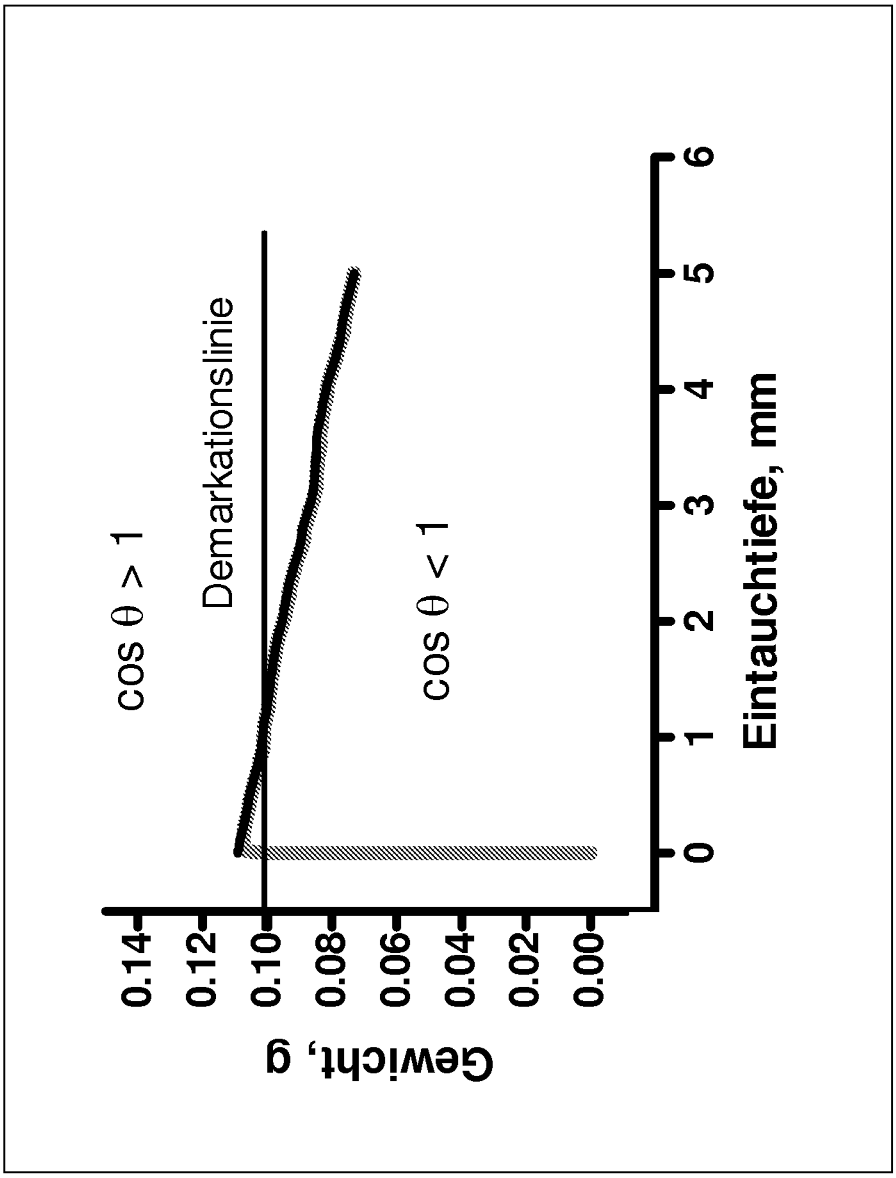
cos θ > 1
Demarkationslinie
cos θ < 1
Gewicht, g
0.14
0.12
0.10
0.08
0.06
0.04
0.02
0.00
0
1
2
3
4
5
6
Eintauchtiefe, mm

Fig. 6

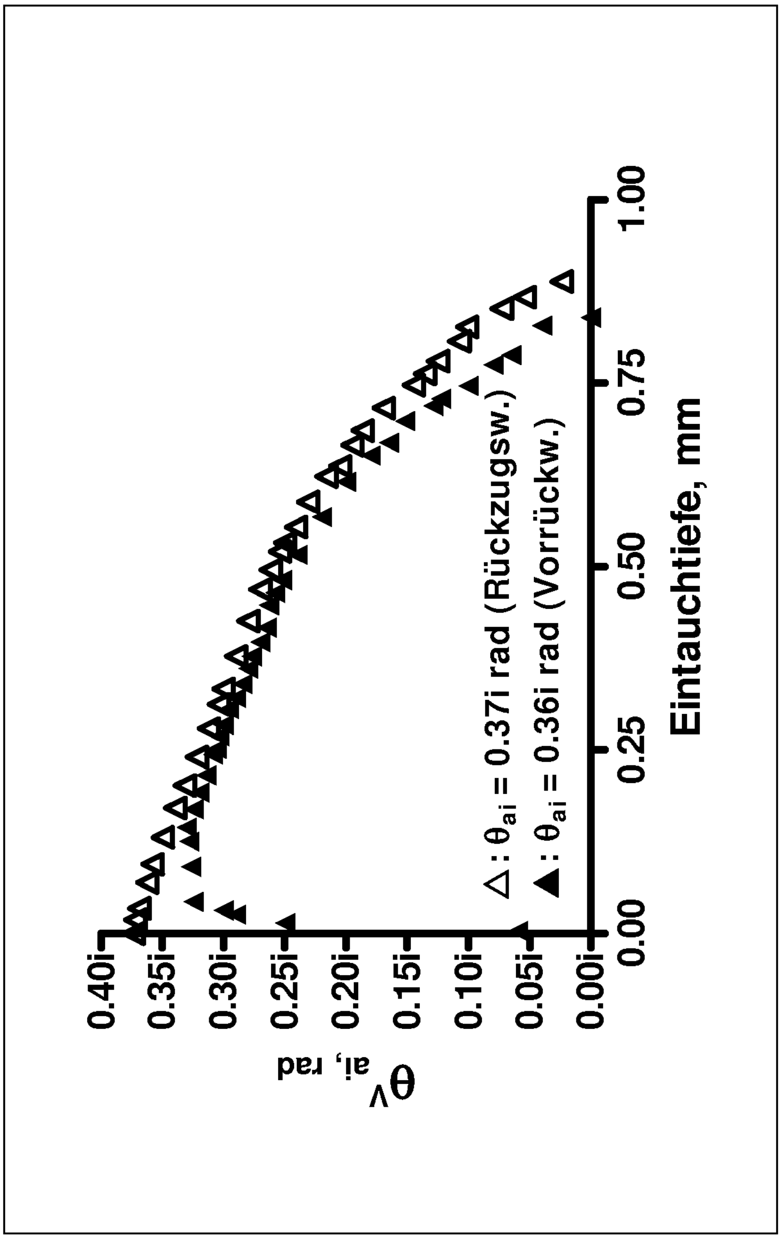
0.40i
0.35i
0.30i
0.25i
0.20i
0.15i
0.10i
0.05i
0.00i
θVai, rad
0.00
0.25
0.50
0.75
1.00
Eintauchtiefe, mm
△ : θai = 0.37i rad (Rückzugsw.)
▲ : θai = 0.36i rad (Vorrückw.)

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2121058 A


### In der Beschreibung aufgeführte Nicht-Patentliteratur


- **MAYS.** A new classification of pore sizes. *Studies in Surface Science and Catalysis,* 2007, vol. 160, 57-62
- **LUDWIG WILHELMY.** *Ann. Phys.,* vol. 119, 177-217